(19) Europäisches Patentamt / European Patent Office / Office européen des brevets

(11) **EP 4 314 807 B1**

(12) **FASCICULE DE BREVET EUROPEEN**

(45) Date de publication et mention de la délivrance du brevet:
**22.01.2025 Bulletin 2025/04**

(21) Numéro de dépôt: **22714189.2**

(22) Date de dépôt: **14.03.2022**

(51) Classification Internationale des Brevets (IPC):
***G01N 33/24*** *(2006.01)*      ***G01N 31/12*** *(2006.01)*

(52) Classification Coopérative des Brevets (CPC):
**G01N 33/24; G01N 31/12**

(86) Numéro de dépôt international:
**PCT/EP2022/056455**

(87) Numéro de publication internationale:
**WO 2022/200091 (29.09.2022 Gazette 2022/39)**

(54) **PROCEDE POUR LA QUANTIFICATION ET LA CARACTERISATION DU CARBONE DANS LES SOLS**

VERFAHREN ZUR QUANTIFIZIERUNG UND CHARAKTERISIERUNG VON KOHLENSTOFF IN BÖDEN

METHOD FOR THE QUANTIFICATION AND CHARACTERIZATION OF CARBON IN SOILS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.03.2021 FR 2103112**

(43) Date de publication de la demande:
**07.02.2024 Bulletin 2024/06**

(73) Titulaire: **IFP Energies nouvelles**
**92500 Rueil-Malmaison (FR)**

(72) Inventeurs:
• **SEBAG, David**
  **92852 RUEIL-MALMAISON CEDEX (FR)**
• **KOWALEWSKI, Isabelle**
  **92852 RUEIL-MALMAISON CEDEX (FR)**
• **LAMOUREUX-VAR, Violaine**
  **92852 RUEIL-MALMAISON CEDEX (FR)**
• **PILLOT, Daniel**
  **92852 RUEIL-MALMAISON CEDEX (FR)**
• **RAVELOJAONA, Herman**
  **92852 RUEIL-MALMAISON CEDEX (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**WO-A1-2015/084784      FR-A1- 3 072 173**

• **F. BEHAR ET AL: "Rock-Eval 6 Technology: Performances and Developments", OIL & GAS SCIENCE AND TECHNOLOGY &NDASH; REVUE DE L&RSQUO;INSTITUT FRANÇAIS DU PÉTROLE, vol. 56, no. 2, 1 March 2001 (2001-03-01), pages 111 - 134, XP055113461, ISSN: 1294-4475, DOI: 10.2516/ogst:2001013**

## Description

## Domaine technique

La présente invention concerne le domaine de la science du sol et des géosciences de l'environnement. Plus particulièrement la caractérisation du carbone contenu dans les formations superficielles, et notamment dans les sols.

Afin de répondre aux enjeux écologiques, ou de respecter certaines législations/directives environnementales, les acteurs de la science du sol et des géosciences de l'environnement (laboratoires de recherche, bureaux d'étude, agences environnementales, exploitants agricoles) sont de plus en plus souvent amenés à mettre en place des protocoles de suivi des impacts des activités humaines sur les stocks de carbone des sols et des éco-agro-systèmes. Ces suivis et ces études d'impact impliquent de pouvoir étudier de larges séries d'échantillons dans des délais relativement courts au regard des méthodologies classiquement employées. De plus, ces méthodes s'accompagnent souvent de contraintes environnementales et sécuritaires qui augmentent les délais et les coûts analytiques, et nécessitent souvent de faire appel à des prestataires spécialisés (par exemple des laboratoires d'analyse).

Les formes organiques du carbone stockées dans les formations superficielles, et notamment dans les sols, représentent un enjeu majeur pour l'agriculture et le climat. Elles jouent en effet un rôle primordial sur la qualité structurelle et la valeur fertilisante des sols, mais interviennent surtout dans le cycle du carbone, en représentant le plus important réservoir de carbone organique à la surface de la Terre. Assurer la sécurité alimentaire des populations et favoriser la séquestration du carbone organique dans les sols nécessite de trouver un compromis entre maintenir des stocks de matière organique suffisamment labiles pour libérer les nutriments nécessaires à la croissance végétale tout en favorisant le stockage de formes de carbone suffisamment résistantes pour atténuer, à long terme, les émissions anthropogéniques de gaz à effet de serre.

Les formes inorganiques de carbone sont de minéralogie variable. Essentiellement représentées par des carbonates et des oxalates de calcium (Ca) dans les sols, elles peuvent parfois intégrer d'autres cations (Fe dans la sidérite, Mg dans la dolomite, etc.), notamment en domaine tropical. Indépendamment de leur rôle dans les cycles biogéochimiques (notamment comme puits de carbone), les formes minérales du carbone posent des problèmes techniques pour l'analyse des formes organiques. En effet, aucune méthode de routine ne permet de caractériser les différentes formes de carbone à l'aide d'une seule et unique mesure, et les approches classiquement employées reposent sur des extractions et des séparations spécifiques (fumigation acide, méthode Walkley and Black, calcimètrie Bernard) suivies d'analyses ciblées à l'aide des équipements scientifiques spécialisés (analyse élémentaire CHN, spectrométrie IR, GC-MS, $^{13}$C-RMN).

On connait aussi des méthodes d'analyse thermique de la matière organique des sols reposant sur des mesures de quantités de composés hydrocarbonés (HC), de monoxyde de carbone (CO) et/ou de dioxyde de carbone ($CO_2$) libérées au cours du temps par un échantillon soumis à une séquence de chauffe en atmosphère inerte puis/ou à une séquence de chauffe en atmosphère oxydante. Ces méthodes ont été développées initialement dans le domaine de l'industrie pétrolière, à des fins de caractérisation de la fraction organique des roches sédimentaires. On connaît ainsi le dispositif ROCK-EVAL® (IFP Energies nouvelles, France), développé par la demanderesse et décrit notamment dans les brevets FR 2227797 (US 3953171) et FR 2472754 (US 4352673), qui comprend un four de pyrolyse distinct d'un four d'oxydation, un détecteur du type à ionisation de flamme (FID) pour détecter les composés hydrocarbonés (HC) et un détecteur du type infrarouge (IR) pour détecter le monoxyde de carbone (CO) et/ou le dioxyde de carbone ($CO_2$). On connait en outre des procédés développés pour des applications particulières du domaine de l'industrie pétrolière, ayant chacun leur propre séquence de températures de chauffe en pyrolyse et/ou de chauffe en atmosphère oxydante. Notamment, on connait la méthode "ROCK-EVAL® BULK ROCK", dédiée plus particulièrement aux échantillons conventionnels de roches mères (Behar et al., 2001). La séquence de chauffe en atmosphère inerte de cette méthode est caractérisée par une température initiale T1 du four de pyrolyse généralement comprise entre 300°C et 350°C, température qui est maintenue pendant une durée prédéterminée de quelques minutes. C'est durant cette phase que sont libérés les hydrocarbures dits « libres » (correspondant en réalité à des hydrocarbures de poids moléculaire léger à lourd) initialement contenus dans l'échantillon de roche. Leur quantité est estimée via la mesure de la surface d'un premier pic, noté $S_1$, de la courbe (aussi appelée thermogramme) représentant la quantité de composés hydrocarbonés libérés pendant la séquence de chauffe en atmosphère inerte. Puis, la température de pyrolyse est augmentée progressivement jusqu'à une température T2, généralement de 650°C. Durant cette phase, on assiste à la volatilisation des composés hydrocarbonés très lourds, ainsi qu'au craquage de la matière organique non volatile (kérogène). La quantité de composés hydrocarbonés libérés durant cette phase de craquage thermique est estimée via la mesure de la surface d'un second pic, noté $S_2$. En parallèle, les quantités de CO et de $CO_2$ sont mesurées et représentées également sous la forme de courbes. Ces courbes présentent deux pics classiquement notés S3CO (respectivement S3CO2), qui est considéré comme correspondant au CO (respectivement $CO_2$) généré par le craquage de la matière organique de l'échantillon pendant la chauffe sous atmosphère inerte, et S3'CO (respectivement S3'CO2) qui est considéré comme correspondant au CO (respectivement $CO_2$) généré par la décomposition thermique des formes carbonatées (notamment la calcite) pendant la chauffe sous atmosphère inerte. Puis le résidu de l'échantillon issu de la chauffe en atmosphère inerte est soumis à une chauffe en atmosphère oxydante : à partir d'une température comprise entre environ 300°C et 400°C, et valant de préférence 300°C, on élève la température du résidu de l'échantillon considéré selon un gradient de température compris entre 20 et 40°C/minute, jusqu'à une température de fin d'oxydation comprise entre 750 et 950°C, et valant de préférence 850°C. Durant cette séquence de chauffe en atmosphère oxydante, les quantités de CO et de $CO_2$ libérées par le résidu de l'échantillon sont mesurées et représentées sous la forme de courbes, conduisant à un pic classiquement noté S4CO (respectivement S4CO2) qui est considéré comme correspondant à la quantité de CO (respectivement $CO_2$) généré par la combustion de la matière organique pendant le cycle d'oxydation. A partir de ces mesures, cette méthode définit un certain nombre de paramètres standards, en particulier le paramètre noté TOC (pour "Total Organic Carbon" en anglais) qui correspond à la teneur en carbone de l'échantillon, déterminée à partir de la quantité totale de HC libérée par l'échantillon et des quantités de CO et de $CO_2$ libérées en-dessous de températures-seuils pendant la phase de pyrolyse et la phase d'oxydation ; et le paramètre noté MinC (pour "Mineral Carbon" en anglais) qui correspond à la teneur en carbone minéral de l'échantillon, déterminée à partir des quantités de CO et de $CO_2$ libérées par l'échantillon au-dessus de températures-seuils pendant la phase de pyrolyse et la phase d'oxydation.

**Technique antérieure**

Les documents suivants seront cités au cours de la description :

Behar F., Beaumont V., De B., Penteado H.L. (2001) Rock-Eval 6 Technology: Performances and Developments, Oil & Gas Science and Technology 56, 111-134.

Disnar, J.R., Guillet, B., Keravis, D., Di-Giovanni, C., Sebag, D., 2003. Soil organic matter (SOM) characterization by Rock-Eval pyrolysis: scope and limitations. Organic Geochemistry 34, 327-343.

Malou, O.P., Sebag, D., Moulin P., Chevallier, T., Badiane-Ndour, N.Y., Thiam, A., Chapuis-Lardy, L. 2020. The Rock-Eval® signature of soil organic carbon in Arenosols of the Senegalese groundnut Basin. How do agricultural practices matter? » Agriculture, Ecosystems & Environment 301: 107030. https://doi.org/10.1016/j.agee.2020.107030.

Pillot, D., Deville, E., Prinzhofer, A., 2014. Identification and Quantification of Carbonate Species Using Rock-Eval Pyrolysis. Oil & Gas Science and Technology - Revue d'IFP Energies nouvelles 69, 341-349.

Sebag, D., Disnar, J.R., Guillet, B., Di Giovanni, C., Verrecchia, E.P., Durand, A., 2006. Monitoring organic matter dynamics in soil profiles by "Rock-Eval pyrolysis": bulk characterization and quantification of degradation. European Journal of Soil Science 57, 344-355.

Sebag, D., Verrecchia, E.P., Cécillon, L., Adatte, T., Albrecht, R., Aubert, M., Bureau, F., Cailleau, G., Copard, Y., Decaëns, T., Disnar, J.-R., Hetényi, M., Nyilas, T., Trombino, L., 2016. Dynamics of soil organic matter based on new Rock-Eval indices. Geoderma 284, 185-203.

Depuis les années 2000, ce type d'analyses a également été utilisé et adapté pour étudier la fraction organique des formations superficielles et notamment des sols.

Par exemple on connait la méthode décrite dans le document (Disnar et al., 2003), dans lequel est proposée une adaptation de la séquence de chauffe en pyrolyse consistant en une température initiale de la séquence de chauffe en atmosphère inerte valant 200°C, au lieu de 300°C dans la méthode "ROCK-EVAL® BULK ROCK" décrite précédemment. Cette température initiale plus basse permet de d'étendre le pic S2 défini précédemment aux températures de craquage des composants organiques les plus thermolabiles. Ces constituants thermolabiles sont en effet beaucoup plus abondants dans les formations superficielles que dans les roches sédimentaires. Pour estimer leur contribution et ainsi évaluer la stabilité thermique de la matière organique, cette méthode définit le paramètre R400 qui mesure la proportion relative du pic S2 correspondant aux températures inférieures à 400°C. Par ailleurs, ce même document met en évidence un écart négatif entre le paramètre TOC tel que défini dans la méthode ROCK-EVAL® BULK ROCK et les teneurs en carbone organique mesurées avec des méthodes normalisées (par exemple des analyses élémentaires) et recommande une correction statistique (c'est-à-dire l'application d'un coefficient correcteur établi sur un panel représentatif d'échantillons de sols) pour corriger le paramètre TOC, défini pour le domaine pétrolier, afin qu'il soit vraiment représentatif de la teneur en carbone organique, notamment pour les échantillons riches en matières organiques peu décomposées par rapport à leurs précurseurs biogéniques (litières, composts, tourbes, etc.).

On connait également le document (Sebag et al., 2006) qui utilise cette séquence de chauffe adaptée, et qui propose en complément une déconvolution du pic S2 pour évaluer le degré de décomposition des constituants organiques. En effet, les thermogrammes des formations superficielles, en particulier des échantillons organiques, présentent une distribution plurimodale dont les modes principaux sont toujours situés dans des intervalles de température particuliers (300-320°C ; 360-380°C ; 420-440°C ; 470-490°C et 540-560°C). La méthode décrite dans ce document consiste à décomposer le pic S2 en cinq distributions gaussiennes élémentaires centrées sur ces modes. Cette déconvolution mathématique revient à estimer la contribution relative au pic S2 que chaque distribution élémentaire considérée comme relative à une classe de constituants définis par leur seule température de craquage. Ces contributions élémentaires sont ensuite utilisées pour calculer de nouveaux paramètres qui mesurent la stabilité thermique globale de la matière organique (R-index) et le degré de décomposition de la fraction thermolabile (I-index). Cependant, la méthode de déconvolution repose sur une approche itérative pour ajuster la position et la surface de chaque distribution élémentaire en fonction de paramètres statistiques fixés arbitrairement, ce qui réduit la reproductibilité de la décomposition.

On connait également le document (Sebag et al., 2016) qui décrit une approche alternative en montrant qu'une intégration des thermogrammes représentatifs de la quantité de HC contenus dans l'échantillon par bandes de température (200-340°C ; 340-400°C ; 400-460°C ; 460-520°C et 520-650°C) aboutit à des résultats comparables à la déconvolution mathématique, et sont, qui plus est, parfaitement reproductibles. Cette approche a été utilisée pour de nombreuses applications, mais souffre de quelques limites intrinsèques. Tout d'abord, elle repose sur la définition de températures-seuils définies empiriquement pour que la surface de chaque bande de température approche au mieux la surface de la distribution élémentaire correspondante obtenue par déconvolution. En outre, ces surfaces et les paramètres qui en découlent ne fournissent qu'une information qualitative dans la mesure où les unes comme les autres correspondent à des proportions relatives ou des rapports de proportions relatives. Enfin, cette approche se limite à l'examen des quantités de HC émises au cours de la phase de pyrolyse, alors que la majorité du carbone est représentée par le CO et le $CO_2$ émis au cours des phases de pyrolyse et d'oxydation. Une autre approche alternative, utilisant la méthode ROCK-EVAL est connue du document FR3072173.

En outre, malgré ces tentatives d'adaptation ou d'amélioration, les méthodes connues ne fournissent pas des valeurs des paramètres standards TOC et MINC qui soient représentatives respectivement de la teneur d'un échantillon en carbone organique et en carbone minéral dans le cas de formations superficielles. En effet, le paramètre TOC (respectivement MINC) de la méthode "ROCK-EVAL® BULK ROCK" mesure la teneur en carbone à partir des quantités de CO et de $CO_2$ émises en-dessous (respectivement au-dessus) de températures-seuils considérées comme des limites thermiques entre les formes organiques et minérales de carbone.

Or cette définition est erronée que ce soit pour des formations carbonatées ou non carbonatées. En effet, comme montré dans le document (Malou et al. 2020) cette définition implique notamment le calcul systématique d'un paramètre MINC, même pour les échantillons sans forme minérale de carbone. Mais plus généralement, comme constaté par la demanderesse, ceci implique que, même dans les échantillons contenant des formes minérales de carbone, une part du MINC provient du craquage thermique des constituants organiques au-dessus des températures-seuils. Enfin, comme montré dans le document (Pillot et al. 2014), certaines formes minérales de carbone (comme la sidérite ou les oxalates) sont susceptibles de se décomposer à des températures inférieures aux températures-seuils utilisées pour les calculs des paramètres TOC et de MINC standards.

La présente invention vise à pallier les inconvénients liés à l'utilisation d'un procédé initialement développé pour le domaine pétrolier pour analyser la matière organique des formations superficielles, et notamment des sols. En particulier, la présente invention vise à obtenir des thermogrammes présentant des pics mieux différenciés et communs à chaque type d'effluents (HC, CO et $CO_2$). Cette invention permet ainsi de mieux discriminer les différentes classes de composés en fonction de leur résistance thermique. Elle conduit également à statuer sur la nature organique et/ou minérale des formes de carbone et ainsi à définir des nouveaux paramètres plus représentatifs de la teneur en carbone organique et en carbone minéral d'échantillons analysés par des méthodes normalisées.

Plus généralement, la présente invention permet une caractérisation rapide et fiable des formes de carbone présentes dans un échantillon pour déterminer si elles contiennent des formes de nature minérale ou non, pour préciser le degré de décomposition et/ou la stabilité thermique de la part de matière organique la plus réactive (liée à l'atome d'hydrogène), et pour quantifier plus précisément les teneurs en carbone (organique versus minéral, thermolabile versus thermostable).

## Résumé de l'invention

La présente invention concerne un procédé pour caractériser et quantifier le carbone présent dans une formation superficielle, à partir d'un échantillon représentatif de ladite formation superficielle, dans lequel on applique au moins les étapes suivantes audit échantillon :

A. on chauffe ledit échantillon selon une première séquence de chauffe sous atmosphère inerte, et on mesure en continu une quantité de composés hydrocarbonés, une quantité de CO et une quantité de $CO_2$ libérés pendant ladite première séquence de chauffe, ladite première séquence de chauffe sous atmosphère inerte comprenant au moins une succession d'au moins six paliers isothermes d'une durée prédéterminée, ladite succession desdits au moins six paliers isothermes comprenant un premier palier isotherme à une première température comprise entre 80 et 200°C (T0), un deuxième palier isotherme à une deuxième température comprise entre 340 et 380°C (T1), un troisième palier isotherme à une troisième température comprise entre 400 et 440°C (T2), un quatrième palier isotherme à une quatrième température comprise entre 450 et 490°C (T3), un cinquième palier isotherme à une cinquième température comprise entre 500 et 540°C (T4), un sixième palier isotherme à une sixième température comprise entre 580 et 650°C (T5), lesdits paliers isothermes étant reliés entre eux par un gradient thermique ;

B. on chauffe un résidu dudit échantillon issu de ladite première séquence de chauffe selon une seconde séquence de chauffe sous atmosphère oxydante, et on mesure une quantité de CO et une quantité de $CO_2$ libérés pendant ladite seconde séquence de chauffe, ladite deuxième séquence de chauffe sous atmosphère oxydante débutant à une température minimale comprise entre 150 et 300°C, se terminant à une température maximale comprise entre 850 et 1200°C, et suivant un gradient thermique

C. on détermine une valeur d'un paramètre SCmin représentatif d'une proportion en carbone minéral par rapport au carbone total dudit échantillon à partir d'un ratio entre ladite quantité de $CO_2$ libéré par ledit résidu dudit échantillon au-delà d'une température intermédiaire de ladite deuxième séquence de chauffe comprise entre 620 et 680°C, et ladite quantité de $CO_2$ libéré par ledit résidu pendant ladite deuxième séquence de chauffe ;

D. on quantifie une teneur en carbone organique et/ou une teneur en carbone minéral dudit échantillon de la manière suivante :

    i. si ladite valeur dudit paramètre SCmin est inférieure ou égale à une valeur seuil prédéfinie dudit paramètre SCmin, ladite teneur en carbone minéral est nulle et ladite teneur en carbone organique est fonction de la totalité desdites quantités de HC, de CO et de CO2 libérés pendant ladite première séquence de chauffe, et desdites quantités de CO et de CO2 libérés pendant ladite deuxième séquence de chauffe jusqu'à ladite température intermédiaire de ladite deuxième séquence de chauffe.

    ii. si ladite valeur dudit paramètre SCmin est supérieure à ladite valeur seuil prédéfinie dudit paramètre SCmin :

    -   on estime une quantité de $CO_2$ libéré par craquage thermique de la matière organique dudit échantillon pendant ladite première séquence de chauffe au-delà de ladite troisième température (T2) à partir de ladite quantité de $CO_2$ libéré pendant ladite première séquence de chauffe jusqu'à ladite troisième température (T2) de ladite première séquence de chauffe, multipliée par un facteur k prédéfini ; et/ou

    -   on détermine ladite teneur en carbone organique et/ou ladite teneur en carbone minéral en fonction desdites quantités de HC, de CO et de $CO_2$ libérés pendant ladite première séquence de chauffe et desdites quantités de CO et de $CO_2$ libérés pendant ladite deuxième séquence de chauffe jusqu'à ladite température intermédiaire de ladite deuxième séquence de chauffe, et en prenant en compte ladite quantité estimée $CO_2$ libéré par craquage thermique de la matière organique dudit échantillon pendant ladite première séquence de chauffe au-delà de ladite troisième température (T2).

Selon une mise en œuvre de l'invention, si ladite valeur dudit paramètre SCmin est supérieure à ladite valeur seuil prédéfinie dudit paramètre SCmin :

-    ladite teneur en carbone organique peut être fonction de la totalité de ladite quantité de HC libérés pendant ladite première séquence de chauffe, de ladite quantité de CO libéré pendant ladite première séquence de chauffe jusqu'à ladite cinquième température (T4) à laquelle on ajoute la moitié de ladite quantité de CO libéré pendant ladite première séquence de chauffe au-delà de ladite cinquième température (T4), de ladite quantité de $CO_2$ libéré pendant ladite première séquence de chauffe jusqu'à ladite troisième température (T2) à laquelle on ajoute ladite quantité estimée de $CO_2$ libéré par craquage thermique de la matière organique dudit échantillon pendant ladite première séquence de chauffe au-delà de ladite troisième température (T2), et desdites quantités de CO et de CO2 libérés pendant ladite deuxième séquence de chauffe jusqu'à ladite température intermédiaire de ladite deuxième séquence de chauffe, et/ou

-    ladite teneur en carbone minéral peut être fonction de ladite quantité de $CO_2$ libéré pendant ladite première séquence de chauffe au-delà de ladite troisième température (T2) de ladite première séquence de chauffe à laquelle on retire ladite quantité estimée de $CO_2$ libéré par craquage thermique de la matière organique dudit échantillon pendant ladite première séquence de chauffe au-delà de ladite troisième température (T2), de ladite moitié de ladite quantité de CO libéré pendant ladite première séquence de chauffe au-delà de ladite cinquième température (T4), et de ladite quantité de $CO_2$ libéré pendant ladite deuxième séquence de chauffe au-delà de ladite température intermédiaire de ladite deuxième séquence de chauffe.

Selon une mise en œuvre de l'invention, ledit facteur k prédéfini peut être compris entre 1.3 et 1.4, et vaut de préférence 1.3724.

Selon une mise en œuvre de l'invention, au moyen d'une pluralité d'échantillons provenant de formations superficielles non carbonatées, on peut appliquer au moins ladite première séquence de chauffe à chacun desdits échantillons de ladite pluralité d'échantillons, on peut mesurer une quantité de $CO_2$ libéré par chacun desdits échantillons pendant ladite première séquence de chauffe, et on peut déterminer ledit facteur k au moyen d'une régression linéaire appliquée entre lesdites quantités de $CO_2$ libérés par ladite pluralité d'échantillons jusqu'à ladite troisième température (T2) de ladite première séquence de chauffe et lesdites quantités de $CO_2$ libérés par ladite pluralité d'échantillons au-delà de ladite troisième température (T2) de ladite première séquence de chauffe.

Selon une mise en œuvre de l'invention, ladite valeur seuil prédéfinie dudit paramètre SCmin peut être comprise entre 0.03 et 0.05 et vaut de préférence 0.04.

Selon une mise en œuvre de l'invention, ladite première température (T0) de la dite première séquence de chauffe peut être comprise entre 80°C et 150°C, et vaut de préférence 150°C.

Selon une mise en œuvre de l'invention, ladite température minimale de ladite deuxième séquence de chauffe peut être égale à ladite première température (T0) de la dite première séquence de chauffe.

Selon une mise en œuvre de l'invention, ladite durée prédéterminée d'un desdits paliers isothermes de ladite première séquence de chauffe peut être comprise entre 3 et 5 minutes.

Selon une mise en œuvre de l'invention, l'un desdits gradients thermiques de ladite première séquence de chauffe peut être compris entre 1 et 50°C.min$^{-1}$, de préférence entre 20 et 25°C.min$^{-1}$.

Selon une mise en œuvre de l'invention, ledit gradient thermique de ladite deuxième séquence de chauffe peut être compris entre 20 et 40°C.min$^{-1}$, de préférence entre 20 et 25°C.min$^{-1}$.

Selon une mise en œuvre de l'invention, si ladite valeur dudit paramètre SCmin est inférieure ou égale à ladite valeur seuil prédéfinie dudit paramètre SCmin :

- ladite teneur en carbone minéral peut être définie par un paramètre MinCfs tel que MinCfs=0 ; et/ou
- ladite teneur en carbone organique peut être définie par un paramètre TOCfs s'exprimant selon une formule du type :

$$TOC\text{fs} = (S2_t \times 0.083) + (S3CO2_t \times \frac{12}{440}) + (S3CO_t \times \frac{12}{280}) + (S4CO2_a \times \frac{12}{440}) + (S4CO_a \times \frac{12}{280})$$

où $S2_t$, $S3CO2_t$, $S3CO_t$ représentent lesdites quantités respectivement de HC, de $CO_2$ et de CO libérés pendant ladite première séquence de chauffe, $S4CO2_a$ et $S4CO_a$ représentent respectivement lesdites quantités de $CO_2$ et de CO libérés pendant ladite deuxième séquence de chauffe et jusqu'à ladite température intermédiaire de ladite deuxième séquence de chauffe.

Selon une mise en œuvre de l'invention, si ladite valeur dudit paramètre SCmin est supérieure à ladite valeur seuil prédéfinie dudit paramètre SCmin :

- ladite teneur en carbone minéral peut être définie par un paramètre MinCfs s'exprimant selon une formule du type :

$$MinC\text{fs} = ([S3CO2_b - S3CO2_c] \times \frac{12}{440}) + (\frac{1}{2} S3CO_b \times \frac{12}{280}) + (S4CO2_b \times \frac{12}{440})$$

où $S3CO2_b$ représente ladite quantité de $CO_2$ libéré pendant ladite première séquence de chauffe au-delà de ladite troisième température (T2), $S3CO2_c$ représente ladite quantité estimée de $CO_2$ libéré par craquage thermique de la matière organique dudit échantillon pendant ladite première séquence de chauffe au-delà de ladite troisième température (T2), $S3CO_b$ représente ladite quantité de CO libéré pendant ladite première séquence de chauffe au-delà de ladite cinquième température (T4), et $S4CO2_b$ représente ladite quantité de $CO_2$ libéré pendant ladite deuxième séquence de chauffe au-delà de ladite température intermédiaire de ladite deuxième séquence de chauffe ; et/ou

- ladite teneur en carbone organique peut être définie par un paramètre TOCfs s'exprimant selon une formule du type :

$$TOC\text{fs} = (S2_t \, x \, 0.083) + \left([S3CO2_a + S3CO2_c] x \frac{12}{440}\right) + \left([S3CO_a + \tfrac{1}{2} S3CO_b] x \frac{12}{280}\right) +$$

$$(S4CO2_a \, x \frac{12}{440}) + (S4CO_a \, x \frac{12}{280})$$

où $S2t$ représente ladite quantité de HC libérés pendant ladite première séquence de chauffe, $S3CO2_a$ représente ladite quantité de $CO_2$ libéré pendant ladite première séquence de chauffe jusqu'à ladite troisième température (T2), $S3CO2_c$ représente ladite quantité estimée de $CO_2$ libéré par craquage thermique de la matière organique dudit échantillon pendant ladite première séquence de chauffe au-delà de ladite troisième température (T2), $S3CO_a$ et $S3CO_b$ représentent lesdites quantités de CO libérés pendant ladite première séquence de chauffe respectivement jusqu'à et au-delà de ladite cinquième température (T4), $S4CO2_a$ et $S4CO_a$ représentent respectivement lesdites quantités de $CO_2$ et de CO libérés pendant ladite deuxième séquence de chauffe et jusqu'à ladite température intermédiaire de ladite deuxième séquence de chauffe.

D'autres caractéristiques et avantages du procédé selon l'invention, apparaîtront à la lecture de la description ci-après d'exemples non limitatifs de réalisations, en se référant aux figures annexées et décrites ci-après.

**Liste des figures**

La figure 1 illustre de manière schématique la séquence de chauffe sous atmosphère inerte selon l'invention.

Les figures 2A et 2B illustrent des thermogrammes obtenus au moyen d'une mise en œuvre de la séquence de chauffe sous atmosphère inerte selon l'invention, obtenus respectivement pour un échantillon de sol non carbonaté et pour un échantillon de sol carbonaté.

Les figures 2C et 2D illustrent des thermogrammes obtenus au moyen d'une séquence de chauffe selon l'art antérieur, obtenus respectivement pour les échantillons de sol non carbonaté et de sol carbonaté des figures 2A et 2B.

Les figures 3A et 3B illustrent des thermogrammes obtenus au moyen d'une mise en œuvre de la séquence de chauffe sous atmosphère oxydante selon l'invention, obtenus respectivement pour l'échantillon de sol non carbonaté des figures 2A et 2C et pour l'échantillon de sol carbonaté des figures 2B et 2D.

Les figures 4A et 4B présentent des histogrammes comparant les valeurs respectivement des teneurs en carbone organique Corg et des teneurs en carbone minéral Cmin déterminées par le procédé selon l'invention et par l'art antérieur pour 9 échantillons de sols.

Les figures 5A et 5B présentent des histogrammes comparant les valeurs respectivement des teneurs en carbone organique Corg et des teneurs en carbone minéral Cmin déterminées par le procédé selon l'invention et par l'art antérieur pour 7 échantillons de tourbes.

**Description des modes de réalisation**

L'invention concerne un procédé pour caractériser et quantifier le carbone (ou autrement dit les formes de carbone) présentes dans une formation superficielle.

Par "formation superficielle" ("superficial deposit" en anglais), on entend une formation continentale ou littorale, meuble ou secondairement consolidée, provenant de la désagrégation mécanique et/ou chimique de roches préexistantes, et formée à l'interface lithosphère/biosphère/atmosphère. On distingue (i) les "formations superficielles allochtones" (comme les colluvions, alluvions, lœss, etc.) qui ont subi ou subissent encore des déplacements proches ou lointains, et ne reposent plus sur leur matériel-parent, et les "formations superficielles autochtones" (comme les arènes, altérites, argiles à silex, etc.) qui ont évolué sur place à partir d'un matériel parent qui constitue encore leur substrat.

Par sol ("soil" en anglais), on entend l'ensemble des couches externes des formations superficielles, dont les propriétés sont directement contrôlées par les actions mutuelles de l'eau, de l'air et des organismes vivants et morts, voire des activités humaines pour les périodes les plus récentes.

Ces termes sont notamment définis dans le document de référence (Dictionnaire encyclopédique de Science du Sol, Mathieu & Lozet, Lavoisier, 2011).

Le procédé selon l'invention requiert de disposer d'au moins un échantillon représentatif de la formation superficielle : cet échantillon peut avoir été prélevé manuellement dans une fosse ou par carottage à l'aide d'une tarière.

Avantageusement, l'échantillon tel que prélevé est tamisé au moyen d'un tamis dont les orifices ont un diamètre de 2 mm, séché à une température inférieure à 40°C, puis broyé jusqu'à obtenir des fragments ayant des dimensions inférieures à 200 $\mu$m.

Le procédé selon l'invention peut être avantageusement mais non limitativement mis en œuvre au moyen du dispositif ROCK-EVAL® (IFP Energies nouvelles, France), tel que décrit dans les brevets FR 2227797 (US 3953171) et FR 2472754 (US 4352673). En effet, le dispositif ROCK-EVAL® comprend au moins :

- un four de pyrolyse en atmosphère non oxydante,

- des moyens de transfert des résidus de pyrolyse dans un four d'oxydation,

- un four d'oxydation en atmosphère oxydante,

- des moyens de mesure de la quantité de composés hydrocarbonés (HC) libérés au cours de la pyrolyse,

- des moyens de mesure du monoxyde de carbone (CO) et du dioxyde de carbone ($CO_2$).

Le procédé peut également être mis en œuvre au moyen d'un unique four de pyrolyse, pouvant fonctionner à la fois en atmosphère non oxydante et en atmosphère oxydante, coopérant avec un dispositif de mesures de la quantité de composés hydrocarbonés libérés au cours de la pyrolyse, et un dispositif de mesure du monoxyde de carbone et du dioxyde de carbone.

Le procédé selon l'invention comprend au moins les étapes suivantes :

**1- Séquence de chauffe sous atmosphère inerte (pyrolyse)**

**2- Séquence de chauffe sous atmosphère oxydante (oxydation)**

**3- Caractérisation et quantification du carbone présent dans l'échantillon**

Les étapes du procédé selon l'invention sont détaillées ci-après de manière non limitative pour un échantillon de sol. Les étapes du procédé selon l'invention peuvent en effet tout aussi bien être appliquées à un échantillon provenant d'une autre couche d'une formation superficielle.

**1. Séquence de chauffe sous atmosphère inerte (pyrolyse)**

Au cours de cette étape, un échantillon de sol est chauffé sous atmosphère inerte (comme par exemple sous un flux d'azote, d'hélium) selon une séquence de températures prédéfinies, variables dans le temps.

Selon l'invention, la séquence de chauffe sous atmosphère inerte comprend au moins une succession de paliers isothermes, chaque palier isotherme ayant une durée prédéterminée, et deux paliers isothermes consécutifs dans cette succession de paliers isothermes étant reliés entre eux par une rampe se présentant sous la forme d'un gradient thermique prédéterminé. Autrement dit, la séquence de chauffe sous atmosphère inerte comprend une succession de paliers isothermes reliés entre eux par une rampe, la durée de chaque palier isotherme pouvant être différente de la durée des autres paliers isothermes, et la pente d'une rampe pouvant être différente de la pente des autres rampes thermiques.

Selon l'invention, la succession de paliers isothermes comprend au moins six paliers isothermes reliés entre eux par cinq rampes. Selon l'invention, la succession de paliers isothermes comprend un premier palier isotherme à une température (notée T0 par la suite) comprise entre 80 et 200°C, un deuxième palier isotherme à une température (notée T1 par la suite) comprise entre 340 et 380°C, un troisième palier isotherme à une température (notée T2 par la suite) comprise entre 400 et 440°C, un quatrième palier isotherme à une température (notée T3 par la suite) comprise entre 450 et 490°C, un cinquième palier isotherme à une température (notée T4 par la suite) comprise entre 500 et 540°C, un sixième palier isotherme à une température (notée T5 par la suite) comprise entre 580 et 650°C.

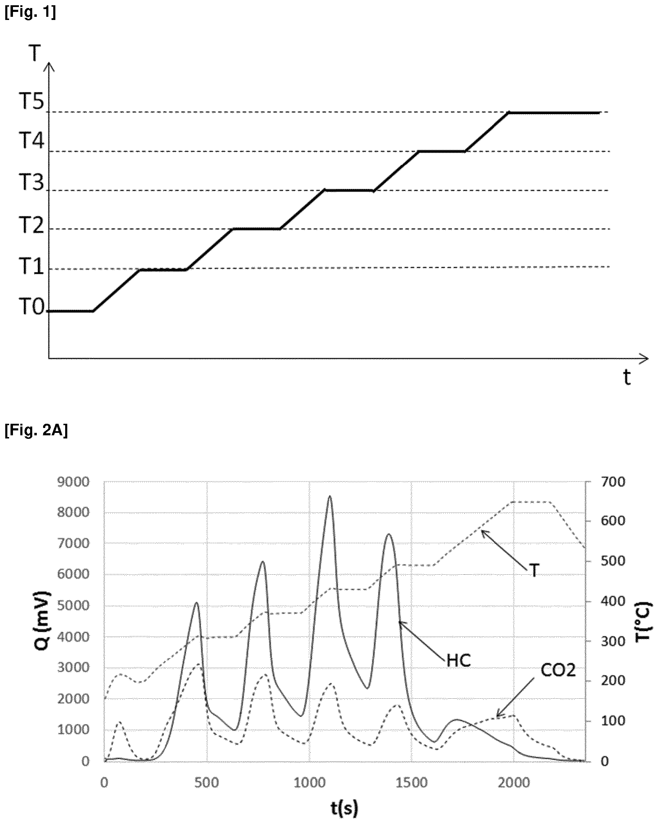

La figure 1 illustre de manière schématique une telle séquence de températures, en représentant l'évolution de la température T en fonction du temps t, la séquence de températures présentant six paliers isothermes aux températures T0, T1, T2, T3, T4 et T5 telles que définies ci-dessus.

Selon l'invention, la durée prédéterminée des paliers isothermes est non nulle (par exemple supérieure à une demi-minute), et peut être préférentiellement comprise entre 3 et 5 minutes. De telles durées permettent de considérer que le craquage des composés ayant une température de craquage proche de la température du palier isotherme est terminé. De manière générale, la durée d'un palier isotherme peut être différente de la durée des autres paliers isothermes dans la séquence de chauffe en atmosphère inerte.

Avantageusement, le gradient thermique entre deux paliers isothermes peut être compris entre 1 et 50°C.min$^{-1}$, de préférence entre 20° et 25°C.min$^{-1}$. De telles valeurs constituent des compromis permettant de débuter le craquage thermique des composés associés au palier isotherme ayant la température la plus élevée parmi ces deux paliers isothermes, tout en limitant la durée de mise en œuvre du procédé. De manière générale, la valeur d'un gradient thermique entre deux paliers isothermes peut être différente des autres gradients thermiques dans la séquence de chauffe en atmosphère inerte.

Selon l'invention, on mesure également, en continu, une quantité de composés hydrocarbonés libérés durant la chauffe sous atmosphère inerte, ainsi que la quantité de $CO_2$ et de CO contenus dans l'effluent résultant de ladite chauffe. Autrement dit, au cours de cette séquence, on mesure en continu la quantité de HC, de CO et de $CO_2$ libérés par l'échantillon par craquage thermique de la matière organique et par la décomposition thermique des minéraux carbonatés. Ainsi, on obtient à l'issue de cette étape appliquée à un échantillon donné, une première courbe représentative de la quantité de composés hydrocarbonés libérés au cours du temps pendant au moins une partie de la phase de pyrolyse, ainsi que deux autres courbes représentatives de la quantité de CO et $CO_2$ libérés au cours du temps, pendant la phase de pyrolyse. La mesure de la quantité de composés hydrocarbonés peut être réalisée au moyen d'un détecteur du type à ionisation de flamme (FID). La mesure de la quantité de CO et $CO_2$ libérés peut être réalisée au moyen d'un détecteur du type infrarouge (IR). A noter que de tels capteurs mesurent un flux de HC, de CO et/ou de $CO_2$ et donnent des valeurs mesurées en millivolts (mV). De manière classique, on peut déterminer une quantité de HC, de CO et/ou de $CO_2$ en déterminant une aire sous la courbe mesurée (éventuellement entre des températures prédéfinies) par ces capteurs, et en divisant cette aire par la masse en mg de l'échantillon. En variante, d'autres moyens de mesure de la quantité de HC, de CO et/ou de $CO_2$ peuvent être utilisés.

De manière générale, cette séquence particulière de chauffe sous atmosphère inerte permet de séparer thermiquement des classes de composés caractérisés par leur température de craquage propre. Ainsi, les composés craquant entre T0 et T1 correspondent à des « composés très labiles thermiquement » qui sont particulièrement abondants dans les tissus biologiques frais ; les composés craquant entre T1 et T2 correspondent aux « composés labiles thermiquement », qui sont majoritaires dans les échantillons organiques comme les litières ou les tourbes ; les composés craquant entre T2 et T3 correspondent aux « composés résistant thermiquement » et majoritaires dans les échantillons organo-minéraux (sols) ou minéraux (alluvions, colluvions) ; les composés craquant entre T3 et T4 correspondent aux « composés réfractaires thermiquement »; et les composés craquant entre T4 et T5 correspondent aux « composés très réfractaires thermiquement » et sont présents en plus grandes proportions dans des résidus de décomposition ou des fractions exogènes, comme les matières organiques pyrogéniques ou pétrogéniques.

Selon une mise en œuvre de l'invention, le premier palier isotherme peut être de préférence à une température comprise entre 80°C et 150°C, de manière à permettre de récupérer les contributions des composés organiques les plus labiles présents dans un échantillon de sol. De préférence, le premier palier isotherme est à une valeur de 150°C, ce qui est une température suffisante pour récupérer les contributions des composés organiques les plus labiles présents dans la plupart des formations superficielles, et notamment les sols.

Selon une mise en œuvre de l'invention, la température maximale T5 de la séquence de chauffe sous atmosphère inerte peut être de préférence 600°C. Une telle température permet d'éviter d'obtenir des courbes de CO et de $CO_2$ présentant des pics incomplets en fin de pyrolyse, notamment lorsque la température maximale atteint 650°C, et en particulier sur des échantillons végétaux (litières, tourbes, composts).

Selon une mise en œuvre de l'invention, la succession de paliers isothermes selon l'invention telle que décrite ci-dessus peut être précédée par une phase de montée en température du four de pyrolyse, qui peut être sous la forme d'un gradient thermique, par exemple compris entre 1 et 50°C.min$^{-1}$, de préférence entre 20° et 25°C.min$^{-1}$, ou de tout autre forme de courbe de montée en température du four de pyrolyse. Cette phase préliminaire de montée en température du four de pyrolyse permet de mettre le four de pyrolyse à la température du premier palier isotherme de la séquence de chauffe en atmosphère inerte selon l'invention. Cette phase préliminaire peut contribuer à débuter le craquage thermique des composés dont la température de craquage est inférieure à la température du premier palier isotherme, notamment dans le cas de tissus biologiques frais.

Selon une mise en œuvre de l'invention, la succession de paliers isothermes selon l'invention telle que décrite ci-dessus peut être suivie par une phase d'abaissement de la température du four de pyrolyse, qui peut être sous la forme d'un gradient thermique, par exemple compris entre -1 et -50°C.min$^{-1}$, de préférence entre -20° et -25°C.min$^{-1}$, ou de tout autre forme de courbe de baisse en température du four de pyrolyse. Cette phase terminale d'abaissement de la température du four de pyrolyse permet, si besoin, de terminer le craquage thermique des composés associés au dernier palier isotherme de la séquence de chauffe sous atmosphère inerte selon l'invention.

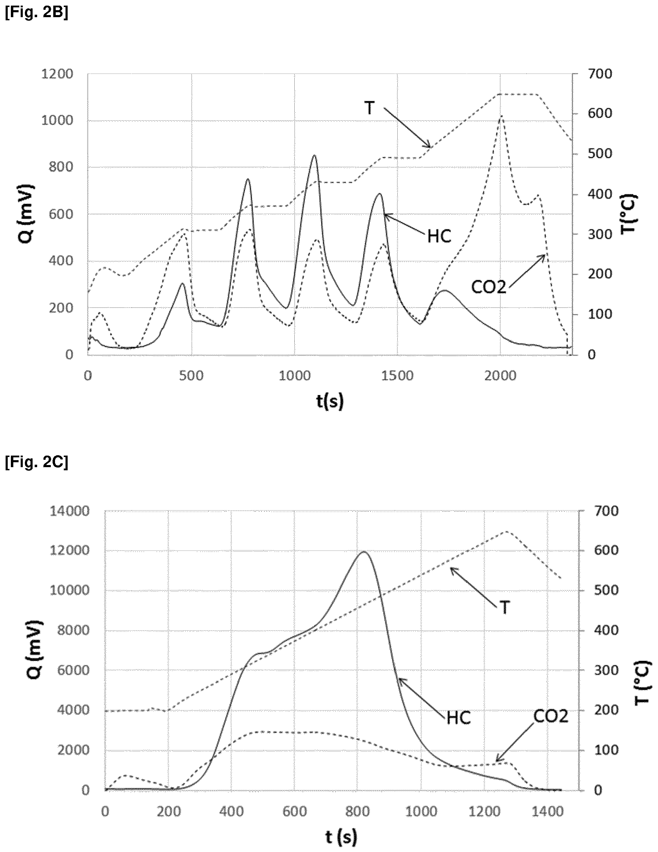

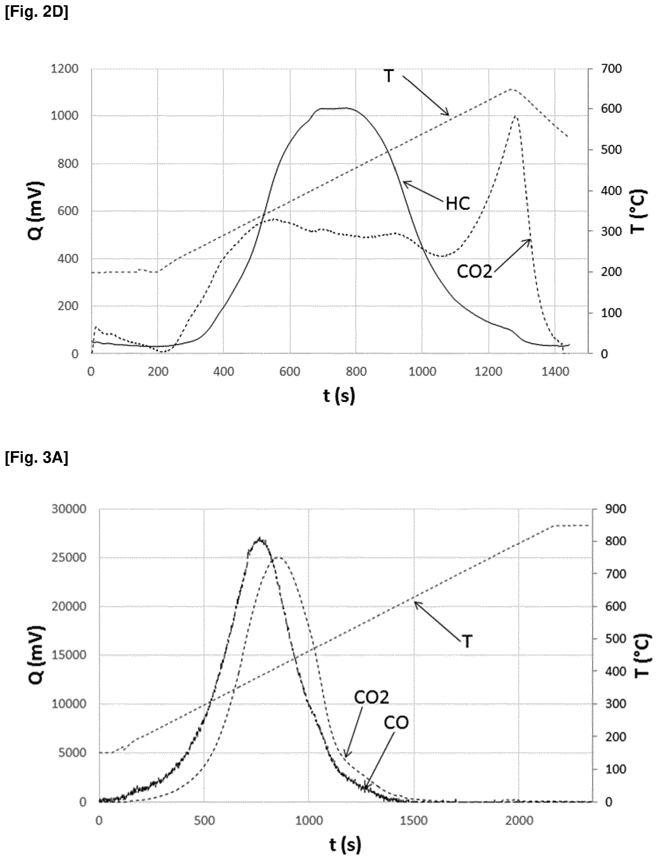

Ainsi, de manière générale, cette séquence particulière de chauffe sous atmosphère inerte permet de séparer thermiquement des classes de composés définis par leur température de craquage spécifique. Ce fractionnement thermique permet notamment d'obtenir des thermogrammes présentant des pics dissociés, et ainsi d'être en mesure de quantifier et d'analyser indépendamment les flux de HC, CO et CO2 libérés chaque classe de composés. Les figures 2A et 2B illustrent des thermogrammes obtenus au moyen de la séquence de chauffe sous atmosphère inerte selon l'invention,

EP 4 314 807 B1

respectivement pour un échantillon de sol non carbonaté et pour un échantillon de sol carbonaté. Les figures 2C et 2D illustrent des thermogrammes obtenus au moyen d'une séquence de chauffe selon l'art antérieur, ne présentant pas de paliers isothermes, obtenus respectivement pour les échantillons de sol non carbonaté et de sol carbonaté des figures 2A et 2B. Plus précisément, sur ces figures, la courbe T représente l'évolution en fonction du temps t de la température du four de pyrolyse pendant cette étape, ou autrement dit la courbe T représente la séquence de chauffe en atmosphère inerte à laquelle sont soumis les échantillons. On peut observer sur cette figure que la séquence de chauffe en atmosphère inerte mise en œuvre pour ces échantillons comprend une phase préliminaire de mise en température du four de pyrolyse jusqu'à une température du premier palier isotherme valant 200°C, ainsi qu'une phase terminale d'abaissement de la température du four de pyrolyse à la fin du dernier palier isotherme qui est à une température de 650°C. Par ailleurs, la courbe HC représente l'évolution de l'intensité Q (en mV) du signal d'un capteur FID mesurant la quantité de composés hydrocarbonés libérés pendant la séquence de chauffe en atmosphère inerte et la courbe CO2 représente l'évolution de l'intensité Q (en mV) du signal d'un capteur IR mesurant la quantité de $CO_2$ libéré pendant la séquence de chauffe en atmosphère inerte. On peut observer sur les figures 2A et 2B que les courbes HC et CO2 présente des pics bien dissociés, contrairement aux courbes des figures 2C et 2D, ce qui démontre que la séquence de chauffe multi-paliers sous atmosphère inerte permet bien un fractionnement thermique des composés contenus dans un échantillon en classes de composés ayant leur température de craquage propre.

A noter qu'une analyse de sensibilité, menée sur un panel diversifié de formations superficielles comprenant des sols et des sédiments récents, montre que la séquence de chauffe sous atmosphère inerte selon l'invention ne modifie pas significativement les quantités de HC, de CO et de $CO_2$ libérées au cours de la pyrolyse. Autrement dit, la quantité totale de HC, de CO et de $CO_2$ est conservée par la séquence de chauffe sous atmosphère inerte selon l'invention. Par contre, la séquence de chauffe multi-paliers selon l'invention permet de mieux dissocier les pics relatifs à des classes de composés distinctes comme discuté ci-dessus.

Par la suite, on utilisera les paramètres définis dans le tableau 1 ci-après. Plus précisément, les paramètres $S2_i$ (respectivement $S3CO2_i$ et $S3CO_{i,}$), avec i variant de 0 à 6 correspondent aux quantités de HC (respectivement $CO_2$ et CO) libérés pendant les gammes de températures $DT_i$ définies de la manière suivante : $DT_0$ = T0- ; T0 < $DT_1$ ≤ T1; T1 < $DT_2$ ≤ T2 ; T2 < $DT_3$ ≤ T3; T3 < $DT_4$ ≤ T4; T4 < $DT_5$ ≤ T5 ; $DT_6$ = T5+ ; et où T0- correspond au premier palier isotherme à la température T0 de la séquence de chauffe en atmosphère inerte, éventuellement précédé d'une phase préliminaire de montée en température du four de pyrolyse ; et où T5+ correspond au dernier palier isotherme à la température T5 de la séquence de chauffe en atmosphère inerte, éventuellement suivi d'une phase terminale d'abaissement de la température du four de pyrolyse.

[Table 1]

| Température | Mesure HC | Mesure $CO_2$ | Mesure CO |
|---|---|---|---|
| $DT_0$ = T0- | $S2_0$ | $S3CO2_0$ | $S3CO_0$ |
| T0 < $DT_1$ ≤ T1 | $S2_1$ | $S3CO2_1$ | $S3CO_1$ |
| T1 < $DT_2$ ≤ T2 | $S2_2$ | $S3CO2_2$ | $S3CO_2$ |
| T2 < $DT_3$ ≤ T3 | $S2_3$ | $S3CO2_3$ | $S3CO_3$ |
| T3 < $DT_4$ ≤ T4 | $S2_4$ | $S3CO2_4$ | $S3CO_4$ |
| T4 < $DT_5$ ≤ T5 | $S2_5$ | $S3CO2_5$ | $S3CO_5$ |
| $DT_6$ = T5+ | $S2_6$ | $S3CO2_6$ | $S3CO_6$ |

**2) Séquence de chauffe sous atmosphère oxydante (oxydation)**

Au cours de cette deuxième étape, le résidu solide de l'échantillon obtenu à l'issue de la séquence de de chauffe sous atmosphère inerte telle que décrite à l'étape 1 ci-dessus est soumis à une oxydation selon un programme de températures prédéfinies, variables dans le temps.

Le programme de températures de la séquence de chauffe sous atmosphère oxydante selon l'invention est le suivant : à partir d'une température (T'min) comprise entre 150°C et 300°C, de préférence comprise entre 150°C et 200°C, et valant de manière préférentielle 150°C ou de manière très préférée étant égale à la température du premier palier isotherme de la séquence de chauffe en atmosphère inerte de manière à pouvoir effectuer des comparaisons, on élève la température du résidu de l'échantillon issu de l'étape 1) selon un gradient de température compris entre 20 et 40°C.min[-1], de préférence compris entre 20° et 25°C.min[-1], jusqu'à une température de fin d'oxydation (T'max) comprise entre 850 et 1200°C, et valant de préférence 900°C qui est une température suffisante pour épuiser le stock de carbone minéral pour la plupart des échantillons de formations superficielles.

Selon l'invention, on mesure, en continu, une quantité représentative de CO et $CO_2$ libérés pendant cette seconde séquence de chauffe. Selon une mise en œuvre de l'invention, cette mesure peut être réalisée au moyen d'un détecteur du type infrarouge (IR). A noter qu'un tel capteur mesure un flux de CO et/ou de $CO_2$, et fournit des valeurs mesurées en millivolts (mV). De manière classique, on détermine une quantité de CO et/ou de $CO_2$ en déterminant une aire sous la courbe mesurée (éventuellement entre des températures prédéfinies) par ce capteur, et on divise cette aire par la masse en mg de l'échantillon. En variante, d'autres moyens de mesure de la quantité de CO et/ou de $CO_2$ peuvent être utilisés.

De manière générale, la gamme de températures préférentielle pour la température initiale de la séquence de chauffe en atmosphère oxydante, plus basse que les températures initiales connues de l'art antérieur (en général 300°C), permet d'éviter des épisodes de combustion instantanée du résidu de l'échantillon au début du cycle d'oxydation.

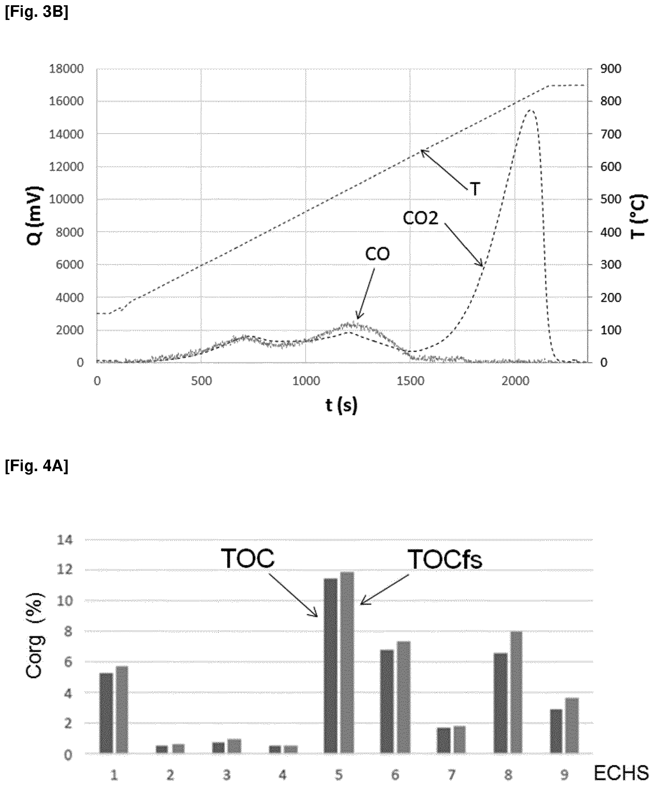

Les figures 3A et 3B illustrent des thermogrammes obtenus au moyen de la séquence de chauffe sous atmosphère oxydante selon l'invention, respectivement pour l'échantillon de sol non carbonaté des figures 2A et 2C et pour l'échantillon de sol carbonaté des figures 2B et 2D. Plus précisément, sur ces figures, la courbe T représente l'évolution en fonction du temps t de la température du four d'oxydation pendant cette étape, ou autrement dit la courbe T représente la séquence de chauffe en atmosphère oxydante à laquelle sont soumis les résidus des échantillons. Par ailleurs, la courbe CO2 (respectivement CO) représente l'évolution de l'intensité QCO2 en mV (respectivement QCO) du signal d'un capteur IR mesurant la quantité de $CO_2$ (respectivement CO) libéré au cours du temps par le résidu issu de l'étape 1) et soumis à la séquence de chauffe sous atmosphère oxydante T. On peut observer sur ces figures les flux de $CO_2$ au-dessus d'une température d'environ 650°C qui sont spécifiques aux sols carbonatés et témoignent de la présence de formes minérales de carbone.

Par la suite, on utilisera les paramètres définis dans le tableau 2 ci-après. Plus précisément, les paramètres $S4CO2_i$ et $S4CO_i$, avec i variant de 0 et 4 correspondent aux quantités de $CO_2$ et CO libérés pendant les gammes de températures $DT'_i$ définies de la manière suivante : $DT'0 \leq T'min$ ; $T'min < DT'1 \leq T'int1$ ; $T'int1 < DT'2 \leq T'int2$ ; $T'int2 < DT'3 \leq T'int3$ et $T'int3 < DT'4 \leq T'max$, où $T'int1$, $T'int2$, et $T'int3$ sont des températures intermédiaires comprises entre $T'min$ et $T'max$ et sont telles que $T'int1$ est comprise entre 420 et 480°C et vaut de préférence 450°C, $T'int2$ est comprise entre 520 et 580°C et vaut de préférence 550°C, et $T'int3$ est comprise entre 620 et 680°C et vaut de préférence 650°C. Ces températures intermédiaires correspondent à des minimas relatifs observés par la demanderesse sur des courbes de CO et de $CO_2$ mesurées pour une pluralité d'échantillons de formations superficielles, et notamment de sols, de nature et d'origine variées. Considérant que, selon la littérature, la température de combustion est une approximation de la stabilité thermique, les flux de CO et CO2 mesurés dans les gammes de température DT'1 , DT'2 et DT'3 peuvent être rapportés à des classes de constituants organiques de stabilité croissante. Considérant que la limite de stabilité thermique de la calcite est proche de T3, les flux de CO2 mesurés dans la gamme de température DT'4 peuvent être rapportés à des formes minérales de carbone.

[Table 2]

| DT'$_0 \leq$ T'min | S4CO2$_0$ | S4CO$_0$ |
|---|---|---|
| T'min < DT'$_1 \leq$ T'int1 | S4CO2$_0$ | S4CO$_1$ |
| T'int1 < DT'$_2 \leq$ T'int2 | S4CO2$_2$ | S4CO$_2$ |
| T'int2 < DT'$_3 \leq$ T'int3 | S4CO2$_3$ | S4CO$_3$ |
| T'int3 <DT'4 $\leq$ T'max | S4CO2$_4$ | S4CO$_4$ |

### 3) Caractérisation et quantification du carbone présent dans l'échantillon

A cours de cette étape, il s'agit de caractériser et de quantifier le carbone présent dans l'échantillon de sol, et notamment de déterminer une teneur en carbone organique et/ou une teneur en carbone minéral. Cette étape comprend au moins les deux sous-étapes détaillées ci-dessous.

### 3.1) Détermination d'un paramètre caractérisant la proportion en carbone minéral dans l'échantillon

Selon l'invention, cette première sous-étape vise à déterminer un paramètre caractérisant la proportion de carbone minéral par rapport au carbone total présent dans l'échantillon considéré, à partir de la quantité de $CO_2$ mesurée à l'étape 2 décrite ci-dessus.

Plus précisément, selon l'invention, on détermine un paramètre noté SCmin, représentatif d'une proportion en carbone minéral par rapport au carbone total dans ledit échantillon à partir d'un ratio entre la quantité de $CO_2$ libéré par le résidu de l'échantillon au-delà de la température intermédiaire $T'int3$ de la séquence de chauffe sous atmosphère oxydante comprise entre 620 et 680°C (définie à l'étape 2), et la totalité de la quantité de $CO_2$ libéré par ce résidu pendant la séquence de chauffe sous atmosphère oxydante. Ainsi, le paramètre SCmin est défini par la proportion de $CO_2$ libéré par décomposition thermique du carbone minéral contenu l'échantillon au cours de la séquence de chauffe en atmosphère oxydante par rapport à la quantité totale de $CO_2$ émise au cours de cette séquence de chauffe en atmosphère.

Selon une mise en œuvre de l'invention, on détermine le paramètre SCmin selon une formule du type :

$$SCmin = \frac{S4CO2_4}{S4CO2_t} \qquad \text{où} \qquad S4CO2_t = \sum_{i=0}^{i=4} S4CO2_i$$

où les $S4CO2_i$, avec i de 1 et 4, sont tels que définis à l'étape précédente. De manière générale, le terme $S4CO2_4$ correspond très majoritairement au $CO_2$ émis par décomposition des espèce minérales carbonatées et $S4CO2_t$ correspond au flux total de $CO_2$ au cours de la phase d'oxydation.

Selon l'invention, on définit une valeur seuil du paramètre SCmin en deçà de laquelle on peut considérer que l'échantillon de sol est dépourvu de forme minérale (c'est-à-dire que l'échantillon est non carbonaté), ou autrement dit, en deçà de laquelle on peut considérer que toute forme de carbone contenue dans cet échantillon est de nature organique.

Selon une mise en œuvre de l'invention, la valeur seuil du paramètre Scmin, notée *SCmin_seuil,* selon laquelle l'échantillon de sol considéré est dépourvu de forme minérale peut être comprise entre 0.03 et 0.05, et vaut de préférence 0.04. Ces valeurs correspondent aux erreurs liées à la mise en œuvre du procédé selon l'invention, dues à l'appareil de mesure lui-même et à la détermination des quantités de $CO_2$ libérées dans les gammes de températures DT'$_i$ définies dans le tableau 2.

Selon une mise en œuvre de l'invention, on peut en outre caractériser le carbone d'un échantillon en définissant quatre classes d'abondance des formes minérales de carbone d'un échantillon, les classes d'abondance pouvant être définies en fonction de la valeur du paramètre SCmin selon l'invention de la manière suivante :

- Si SCmin < 0.04 , l'échantillon ne comporte pas de forme minérale de carbone ;

- Si 0.04 < SCmin < 0.2 : l'échantillon comporte des formes minérales de carbone sous forme de traces ;

- Si 0.2 < SCmin < 0.6 : l'échantillon comporte des formes minérales de carbone ;

- Si SCmin > 0.6 : l'échantillon comporte des formes minérales de carbone en abondance.

**3.2) Détermination d'une teneur en carbone organique et/ou une teneur en carbone minéral**

Au cours de cette étape, on détermine une teneur en carbone organique et/ou une teneur en carbone minéral à partir des quantités de HC, de CO et de $CO_2$ mesurées lors des étapes 1) et 2) décrites ci-dessus, et en fonction de la valeur du paramètre SCmin décrit à la sous-étape 3.1) précédente.

Selon une mise en œuvre de l'invention, on définit des nouveaux paramètres, notés TOCfs et MinCfs par la suite, spécifiques aux séquences de chauffe sous atmosphère inerte et sous atmosphère oxydante selon l'invention et dédiés aux échantillons provenant d'une formation superficielle. Les paramètres TOCfs et MinCfs selon l'invention représentent respectivement la teneur en carbone organique et la teneur en carbone minéral d'un échantillon d'une formation superficielle, contrairement aux paramètres classiques TOC et MinC définis dans l'art antérieur, qui ne sont pas adaptés à des échantillons de formations superficielles. De plus, selon l'invention, les formules pour déterminer les paramètres TOCfs et MinCfs ainsi définies sont fonction de la valeur du paramètre SCmin par rapport à la valeur seuil *SCmin_seuil* définie à la sous-étape précédente.

Selon l'invention, on définit deux cas possibles en fonction de la valeur du paramètre SCmin :

a) Premier cas : Scmin $\leq$ *SCmin_seuil*

Selon l'invention, si la valeur du paramètre SCmin est inférieure à la valeur seuil *SCmin_seuil* définie à la sous-étape précédente (c'est-à-dire si l'échantillon considéré est non carbonaté), la teneur en carbone minéral est nulle et la teneur en carbone organique est fonction de la totalité des quantités de HC, de CO et de $CO_2$ libérés pendant la séquence de chauffe sous atmosphère inerte, et des quantités de CO et de $CO_2$ libérés pendant la séquence de chauffe sous atmosphère oxydante jusqu'à la température intermédiaire T'int3 définie précédemment.

Ainsi, contrairement à l'enseignement de l'art antérieur qui calculait un paramètre MinC non nul même dans le cas d'une absence de carbone minéral dans l'échantillon, la présente invention permet de déterminer une teneur en carbone minéral représentative de la teneur en carbone minéral réelle d'un échantillon d'une formation superficielle sans forme minérale de carbone. De plus, la teneur en carbone organique selon l'invention est déterminée en prenant en compte, contrairement à l'art antérieur, la totalité des quantités de CO et de $CO_2$ libérés pendant les séquences de chauffe en atmosphère inerte et en atmosphère oxydante. En effet, la détermination de la teneur en carbone organique selon l'art antérieur ne considérait que les quantités de CO et $CO_2$ libérés en deçà de la température T2 de la séquence de chauffe sous atmosphère inerte définie à l'étape 1), la moitié de la quantité de CO libéré au-delà de la température T2 de la séquence de chauffe sous atmosphère inerte définie à l'étape 1, et les quantités en CO et $CO_2$ libérés en deçà de la température intermédiaire T'int3 de la séquence de chauffe sous atmosphère oxydante définie à l'étape 2) de la séquence de chauffe sous atmosphère oxydante.

Selon une mise en œuvre de l'invention, si la valeur du paramètre SCmin est inférieure à la valeur seuil *SCmin_seuil* définie à la sous-étape précédente, on peut en outre définir des paramètres MinCfs et TOCfs, représentant respectivement la teneur en carbone minéral et la teneur en carbone organique d'un échantillon d'une formation superficielle, selon les formules suivantes :

- *MinC*fs = 0 *;*

- *TOCfs = PCfs + RC*fs *,* où PCfs est un paramètre représentatif de la teneur en carbone organique pyrolysé d'une formation superficielle et RCfs est un paramètre représentatif de la teneur en carbone organique résiduelle d'une formation superficielle, ces teneurs étant définies par des formules spécifiques aux séquences de chauffe sous atmosphère inerte et sous atmosphère oxydante selon l'invention et pouvant s'écrire de la manière suivante :

$$PC\text{fs} \; = \; (S2_t \; x \; 0.083) \; + \; (S3CO2_t \; x \frac{12}{440}) \; + \; (S3CO_t \; x \frac{12}{280})) \; ;$$

$$RC\text{fs} \; = \; (S4CO2_a \, x \frac{12}{440}) \; + \; (S4CO_a \, x \frac{12}{280}) \; ;$$

Avec :

$$S2_t = \sum_{i=0}^{i=6} S2_i \; ; S3CO2_t = \sum_{i=0}^{i=6} S3CO2_i \; ; S3CO_t = \sum_{i=0}^{i=6} S3CO_i \; ;$$

$$S4CO2_a = \sum_{i=0}^{i=3} S4CO2_i \text{ et } S4CO_a = \sum_{i=0}^{i=3} S4CO_i \; .$$

b) Deuxième cas : Scmin > *SCmin_seuil*

Selon l'invention, si la valeur du paramètre SCmin est supérieure à la valeur seuil *SCmin_seuil* définie à la sous-étape précédente (c'est-à-dire si l'échantillon considéré est carbonaté), on estime préalablement une quantité de $CO_2$ libéré par craquage thermique de la matière organique contenue dans l'échantillon pendant la séquence de chauffe sous atmosphère inerte au-delà de la température T2 définie à l'étape 1) ci-dessus.

Selon l'invention, cette quantité de $CO_2$ libéré par craquage thermique de la matière organique pendant la phase de pyrolyse au-delà de la température T2, notée $S3CO2_c$ par la suite, est estimée à partir de la quantité de $CO_2$ libéré pendant la séquence de chauffe sous atmosphère inerte jusqu'à la température T2, notée $S3CO2_a$ par la suite, multipliée par un facteur k prédéfini, ce qui peut s'écrire selon une formule du type :

$$S3CO2_c = k \; x \; S3CO2_a$$

Selon une première mise en œuvre de l'invention, la valeur du facteur k prédéfini est compris entre 1.3 et 1.4, et vaut de préférence 1.3724.

Selon une deuxième mise en œuvre de l'invention, la valeur du facteur k prédéfini peut être déterminée de manière expérimentale, au moyen d'une pluralité (au moins 10, de préférence au moins 50) d'échantillons provenant de formations superficielles non carbonatées, et pour lesquels on applique au moins la séquence de chauffe sous atmosphère inerte selon l'invention à chacun des échantillons de la pluralité d'échantillons, on mesure en continu une quantité de $CO_2$ libéré par chacun des échantillons pendant cette séquence de chauffe sous atmosphère inerte, et on détermine le facteur k au moyen d'une régression linéaire appliquée entre les quantités de $CO_2$ libérés par la pluralité d'échantillons jusqu'à la température T2 de la séquence de chauffe sous atmosphère inerte définie à l'étape 1) et les quantités de $CO_2$ libérés par la pluralité d'échantillons au-delà de cette température T2. En effet, les échantillons de formations superficielles sans forme minérale de carbone présentent une propriété remarquable : les quantités de $CO_2$ émises au cours des séquences de chauffe en atmosphère inerte au-dessus et en-dessous de la température limite T2 sont linéairement corrélées. Autrement dit, la quantité totale de $CO_2$ émise à une température supérieure à la température limite T2 vaut k fois la quantité totale de $CO_2$ émise à une température inférieure à la température limite T3, ce qui peut s'exprimer selon une formule du type : $\sum_{i=3}^{i=6} S3CO2_i = k \times \sum_{i=0}^{i=2} S3CO2_i$ où k est établi pour des échantillons présentant un SCmin ≤ *SCmin_seuil.* Selon cette mise en œuvre de l'invention, la valeur de ce facteur k, prédéfinie à partir d'une pluralité d'échantillons sans forme minérale, peut être généralisée au cas d'échantillons avec des formes minérales de carbone (c'est-à-dire si SCmin est supérieur à la valeur seuil *SCmin_seuil*) . En effet, considérant que la source du carbone organique est en première approximation comparable pour les sols non carbonatés et les sols carbonatés, il semble raisonnable de considérer que la répartition des composés labiles par rapport aux composés thermiquement stables au-dessus des températures seuils sont comparables pour les deux types d'échantillons.

Dans le cas de d'un échantillon présentant des formes minérales de carbone, la demanderesse a montré que les quantités totales de $CO_2$ libérées au-dessus de la température T2 de la phase de pyrolyse (c'est-à-dire le paramètre $S3CO_b$) cumulent les flux issus du craquage thermique de la matière organique (c'est-à-dire le paramètre $S3CO_c$) et ceux issus de la décomposition thermique de ces formes minérales. Par ailleurs, plus les teneurs en carbone minéral sont élevées, plus la contribution des formes organiques thermiquement stables est négligeable.

Selon l'invention, si la valeur du paramètre SCmin est supérieure à la valeur seuil *SCmin_seuil* définie à la sous-étape précédente, on détermine une teneur en carbone organique et/ou en carbone minéral au moyen des quantités de HC, de CO, et de CO2 mesurées pendant la séquence de chauffe en atmosphère inerte et des quantités de CO et de CO2 mesurées pendant la séquence de chauffe en atmosphère oxydante, et en prenant en compte la quantité $S3CO2_c$ de $CO_2$ libéré par craquage thermique de la matière organique de l'échantillon pendant la séquence de chauffe sous atmosphère inerte au-delà de la température T2, préalablement estimée tel que décrit ci-dessus.

Selon une mise en œuvre de l'invention, si la valeur du paramètre SCmin est supérieure à la valeur seuil *SCmin_seuil* définie à la sous-étape précédente, la teneur en carbone organique peut être déterminée à partir de la totalité de la quantité de HC libérés pendant la séquence de chauffe sous atmosphère inerte, de la quantité de CO libéré pendant la séquence de chauffe sous atmosphère inerte jusqu'à la température T4 à laquelle on ajoute la moitié de la quantité de CO libéré pendant la séquence de chauffe sous atmosphère inerte au-delà cette température T4, de la quantité de $CO_2$ libéré pendant la séquence de chauffe sous atmosphère inerte jusqu'à la température T2 à laquelle on ajoute la quantité estimée de $CO_2$ libéré par craquage thermique de la matière organique pendant la séquence de chauffe sous atmosphère inerte au-delà de la température T2, et des quantités de CO et de $CO_2$ libérés pendant la séquence de chauffe sous atmosphère oxydante jusqu'à la température intermédiaire T'int3 prédéfinie à l'étape 2).

Selon une mise en œuvre de l'invention, si la valeur du paramètre SCmin est supérieure à la valeur seuil *SCmin_seuil* définie à la sous-étape précédente, on peut définir un paramètre TOCfs représentant la teneur en carbone organique d'un échantillon d'une formation superficielle selon une formule du type :

*TOC*fs = *PC*fs + *RCfs* , où PCfs est un paramètre représentatif de la teneur en carbone organique pyrolysé d'un échantillon d'une formation superficielle et RCfs est un paramètre représentatif de la teneur en carbone organique résiduelle d'une formation superficielle, ces teneurs étant définies par des formules spécifiques aux séquences de chauffe sous atmosphère inerte et sous atmosphère oxydante selon l'invention, et pouvant s'écrire de la manière suivante :

$$PC\text{fs} = (S2_t \times 0.083) + \left([S3CO2_a + S3CO2_c] \times \frac{12}{440}\right) + \left([S3CO_a + \frac{1}{2} S3CO_b] \times \frac{12}{280}\right)$$

$$RC\text{fs} = (S4CO2_a \times \frac{12}{440}) + (S4CO_a \times \frac{12}{280})$$

Et où

$$S2_t = \sum_{i=0}^{i=6} S2_i \text{ ;}$$

$$S3CO2_a = \sum_{i=0}^{i=2} S3CO2_i \text{ ;} \quad S3CO2_b = \sum_{i=3}^{i=6} S3CO2_i \text{ ;} \quad S3CO2_c = k \times S3CO2_a$$

$$S3CO_a = \sum_{i=0}^{i=4} S3CO_i \text{ ;} \quad S3CO_b = \sum_{i=5}^{i=6} S3CO_i \text{ ;}$$

$$S4CO2_a = \sum_{i=0}^{i=3} S4CO2_i \text{ ;} \quad S4CO_a = \sum_{i=0}^{i=3} S4CO_i$$

Selon une mise en œuvre de l'invention, si la valeur du paramètre SCmin est supérieure à la valeur seuil *SCmin_seuil* définie à la sous-étape précédente, la teneur en carbone minéral peut être déterminée à partir de de la quantité de $CO_2$ libéré pendant la séquence de chauffe sous atmosphère inerte au-delà de la température T2 à laquelle on retire la quantité estimée de $CO_2$ libéré par craquage thermique de la matière organique pendant la séquence de chauffe sous atmosphère inerte au-delà de la température T2, de la moitié de la quantité de CO libéré pendant la séquence de chauffe sous atmosphère inerte au-delà de la température T4, et de la quantité de $CO_2$ libéré pendant la séquence de chauffe sous atmosphère oxydante au-delà de la température intermédiaire T'int3.

Selon une mise en œuvre de l'invention, si la valeur du paramètre SCmin est supérieure à la valeur seuil *SCmin_seuil* définie à la sous-étape précédente, on peut définir le paramètre MinCfs représentant la teneur en carbone minéral d'un échantillon d'une formation superficielle selon une formule du type :

$$MinCfs = ([S3CO2_b - S3CO2_c]x\frac{12}{440}) + (½\,S3CO_b x\frac{12}{280}) + (S4CO2_b\,x\frac{12}{440})$$

avec

$$S3CO2_b = \sum_{i=3}^{i=6} S3CO2_i \; ; S3CO2_c = k\,x\,S3CO2_a \; ;$$

$$S3CO_b = \sum_{i=5}^{i=6} S3CO_i \, ; S4CO2_b = \sum_{i=3}^{i=4} S4CO2_i \, .$$

Ainsi, les définitions des teneurs en carbone minéral et en carbone organique selon l'invention tiennent compte des formes de carbone présentes de la manière suivante : (i) toutes les formes de carbone sont organiques dans les échantillons tels que SCmin ≤ *SCmin_seuil* et (ii) les autres classes d'abondance de carbone minéral comprennent une part de carbone organique thermiquement stable à laquelle s'ajoute une part de carbone minéral, variable en nature comme en proportion.

Ainsi, à l'issue de ces deux sous-étapes, on obtient une caractérisation et une quantification fiable du carbone présent dans un échantillon d'une formation superficielle, via la détermination d'un paramètre SCmin représentant une proportion en carbone minéral dans l'échantillon, et la détermination de la teneur en carbone organique et/ou en carbone minéral de l'échantillon, par exemple au moyen de paramètres TOCfs et MinCfs définis spécifiquement pour des formation superficielles carbonatées et non carbonatées.

### 3.3) Détermination du statut thermique des formes organiques de carbone

Au cours de cette sous-étape, qui est optionnelle, il s'agit de déterminer le statut thermique des formes organiques de carbone présentes dans l'échantillon. Par statut thermique des formes organiques de carbone présentes dans l'échantillon, on entend un indicateur de la répartition des différentes classes de composés présentes dans l'échantillon et définies chacune par leur température de craquage.

La matière organique d'un sol est un mélange hétérogène complexe comprenant des constituants de nature et d'origine diverses : résidus de la décomposition graduelle des constituants biogéniques les plus labiles, particules libres et constituants impliqués dans les complexes organo-minéraux, constituants pyrogéniques ou pétrogéniques. Le procédé de fractionnement thermique selon l'invention ne permet pas de séparer ces constituants spécifiques. En revanche, il permet de mesurer les contributions des classes de composés définies chacune par leur température de craquage. La stabilité thermique étant considérée comme une variable liée à la stabilité biogéochimique (i.e. la résistance à la décomposition par les microorganismes), les contributions des classes de composés ainsi définies peuvent être utilisées pour décrire l'hétérogénéité de la matière organique des sols.

Selon une première réalisation de cette variante de l'invention comprenant une sous-étape de détermination du statut thermique des formes de carbone de l'échantillon, on peut définir un indice de décomposition ID et un indice de stabilité IS à partir des contributions des classes de composés définies aux étapes 1 et 2 précédentes selon des formules du type :

- Indice de décomposition

$$ID = \log\,[S2_1 + S2_2]\,/\,S2_3$$

- Indice de stabilité

$$IS \ = \ [S2_3 \ + \ S2_4 \ + \ S2_5 \ + \ S2_6] \,/\, 100$$

Ainsi, ces deux indices sont directement liés à la fraction la plus réactive du carbone organique (c'est-à-dire la part de carbone pyrolysé sous forme de composés hydrocarbonés).

L'indice de stabilité IS mesure les contributions relatives des classes de composés thermiquement stables ($S2_3$, $S2_4$, $S2_5$ et $S2_6$) qui sont particulièrement abondantes dans les formations superficielles et les couches profondes des sols, par opposition aux classes de composés plus labiles ($S2_1$ et $S2_2$) et plus abondantes dans les tissus végétaux peu décomposés présents dans les couches organiques et les couches superficielles des sols. L'indice de décomposition ID mesure le rapport entre ces classes de composés les plus labiles ($S2_1$ et $S2_2$) et la classe de composés intermédiaire ($S2_3$) qui est particulièrement abondante dans les couches organiques et organo-minérales des sols. De manière générale, l'indice de décomposition ID mesure le degré de transformation de la matière organique au fur et à mesure que les composés des classes de composés les plus labiles sont décomposés et que les composés des classes de composés les plus stables s'accumulent.

Selon une deuxième réalisation de cette variante de l'invention comprenant une sous-étape de détermination du statut thermique des formes de carbone de l'échantillon, on peut déterminer le statut thermique des formes de carbone d'un échantillon à partir des contributions relatives des différentes classes de composés d'un échantillon pour calculer une teneur en carbone organique thermiquement labile (c'est-à-dire résultant du craquage thermique et de la combustion en-dessous de T3) et une teneur en carbone organique thermiquement stable (c'est-à-dire résultant du craquage thermique et de la combustion en-dessous de T3).

Selon une mise en œuvre de l'invention, on peut déterminer au moins un des paramètres caractérisant le statut thermique de l'échantillon suivants :

- une teneur en carbone organique pyrolysé thermiquement labile, notée COPL, définie selon une formule du type :

$$COPL\,(\%C) \ = \ (S2_L \ x \ 0.083) \ + \ ([KCOPL * S3CO2_L] \ x \frac{12}{440}) \ + \ (S3CO_L \ x \frac{12}{280})$$

avec KCOPL compris entre 1 et 2, et valant préférentiellement 1.3359.

- une teneur en carbone organique pyrolysé thermiquement stable, notée COPS, définie selon une formule du type :

$$COPS\,(\%C) \ = \ (S2_S \, x \ 0.083) \ + \ ([KCOPS * S3CO2_L] x \frac{12}{440}) \ + \ ([\tfrac{1}{2}\,S3CO_S] x \frac{12}{280})$$

avec KCOPS compris entre 0.1 et 1, et valant préférentiellement 0.6274.

- une teneur en carbone organique résiduel thermiquement labile, notée CORL, définie selon une formule du type :

$$CORL\,(\%C) \ = \ (S4CO2_L \ x \frac{12}{440}) \ + \ (S4CO_L \ x \frac{12}{280})$$

- une teneur en carbone organique résiduel thermiquement stable, notée CORS, définie selon une formule du type :

$$CORS\,(\%C) \ = \ (S4CO2_S \ x \frac{12}{440})$$

où

$$S2_L = \sum_{i=0}^{i=3} S2_i\,; \qquad S2_S = \sum_{i=4}^{i=6} S2_i$$

$$S3CO2_L = \sum_{i=0}^{i=3} S3CO2_i\,;\; S3CO2_S = \sum_{i=4}^{i=6} S3CO2_i\,;$$

$$S3CO_L = \sum_{i=0}^{i=4} S3CO_i\,;\; S3CO_S = \sum_{i=5}^{i=6} S3CO_i\,;$$

$$S4CO2_L = \sum_{i=0}^{i=1} S4CO2_i \;\; ; S4CO2_S = \sum_{i=2}^{i=3} S4CO2_i \;\;\;\; ;$$

$$S4CO_L = \sum_{i=0}^{i=1} S4CO_i \;\; ; S4CO_S = \sum_{i=2}^{i=3} S4CO_i \;.$$

Ces teneurs partielles sont particulièrement utiles pour comprendre la dynamique de la matière organique dans les sols car elles permettent de comparer les stocks totaux de carbone organique avec les contributions des différentes formes de carbone, et d'effectuer un suivi de l'évolution de ces stocks au cours du temps. Elles permettent notamment d'explorer les relations entre les stocks en carbone organique thermiquement labile et en en carbone organique thermiquement stable qui sont directement corrélés dans les sols, ce qui indique que le transfert d'un stock à l'autre s'opère effectivement dans les sols.

**Exemples**

Les caractéristiques et avantages du procédé selon l'invention apparaîtront plus clairement à la lecture de l'exemple d'application ci-après.

Le procédé selon l'invention a été appliqué à une série de 9 échantillons de sols distincts, notés par la suite ECHSi avec i variant de 1 à 9, les échantillons ECHS2, ECHS3, ECHS8, ECHS9 provenant de sols carbonatés, et les échantillons ECHS1, ECHS4, ECHS5, ECHS6, ECHS7 provenant de sols non carbonatés.

Le procédé selon l'invention a été en outre appliqué à une série de 7 échantillons de tourbes distinctes, notés par la suite ECHTi avec i=1 à 7 par la suite, provenant d'une même séquence tourbeuse et datés de 340, 2850, 4540, 5220, 6050, 8850 et 9640 ans.

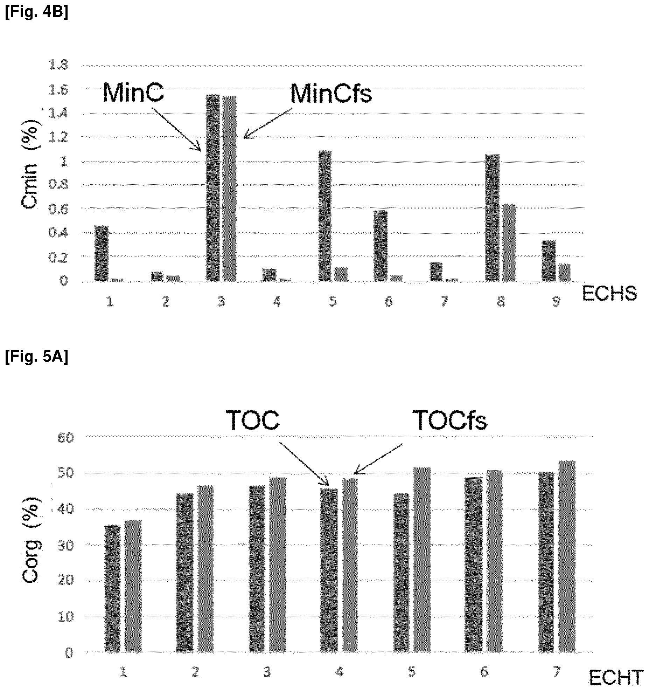

La figure 4A (respectivement la figure 4B) présente un histogramme comparant les valeurs des teneurs en carbone organique Corg (respectivement en carbone minéral Cmin) déterminées par le procédé selon l'invention et par l'art antérieur pour chacun des 9 échantillons de sols ECHS1 à ECHS9. Plus précisément, la figure 4A (respectivement la figure 4B) présente les valeurs (barres de droite en gris clair) du paramètre TOCfs (respectivement MinCf) selon l'invention et les valeurs (barres de gauche en gris foncé) du paramètre TOC (respectivement MinC) selon l'art antérieur pour chacun de ces 9 échantillons. De manière générale, les valeurs de TOCfs sont globalement plus élevées (environ de 8%) que les valeurs de TOC selon l'art antérieur, ce qui correspond à la proportion de carbone organique considéré à tort comme minéral dans l'art antérieur. On peut aussi observer que les valeurs de MinCfs sont nettement plus basses que les valeurs MinC pour les échantillons de sols non carbonatés pour lesquels la valeur de MinC selon l'art antérieur correspond à des flux de CO et de $CO_2$ émis au cours de la pyrolyse et de l'oxydation de formes organiques de carbone. On peut également observer que les valeurs de MinCfs sont plus proches, voire identiques à celle de MinC pour les échantillons de sols carbonatés pour lesquels la proportion de carbone organique comptabilisé à tort dans le MinC selon l'art antérieur est moins importante que dans les sols non carbonatés, voire négligeable si les formes de carbone organique sont entièrement pyrolysées et oxydées en dessous des températures-seuils utilisées pour calculer MINC selon l'art antérieur.

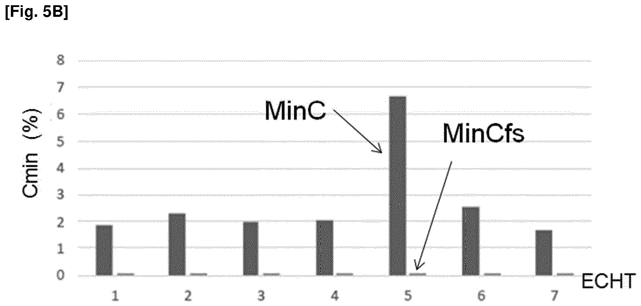

La figure 5A (respectivement la figure 5B) présente un histogramme comparant les valeurs des teneurs en carbone organique Corg (respectivement en carbone minéral Cmin) déterminées par le procédé selon l'invention et par l'art antérieur pour chacun des 7 échantillons de tourbes ECHT1 à ECHT7. Plus précisément, la figure 5A (respectivement la figure 5B) présente les valeurs (barres de droite en gris clair) du paramètre TOCfs (respectivement MinCf) selon l'invention et les valeurs (barres de gauche en gris foncé) du paramètre TOC (respectivement MinC) selon l'art antérieur pour chacun de ces 7 échantillons. On peut observer sur cette figure que les valeurs de TOC (respectivement MinC) et de TOCfs (respectivement MinCfs) sont nettement plus élevées (respectivement basses) puisque, pour ces échantillons organiques, la quasi-totalité du carbone pris en compte pour le calcul du MINC selon l'art antérieur provient en fait de la pyrolyse et de l'oxydation de composés organiques.

Ainsi, la présente invention permet la caractérisation et la quantification fiable des formes de carbone présentes dans un échantillon d'une formation superficielle, que la formation superficielle soit carbonatée ou non. En particulier, la présente invention définit des séquences de chauffe en atmosphère inerte et en atmosphère oxydante appropriées aux formations superficielles, ainsi que des teneurs en carbone organique et/ou en carbone minéral prenant en compte une quantité estimée de $CO_2$ libéré par craquage thermique de la matière organique de l'échantillon pendant la séquence de chauffe en atmosphère inerte au-delà de la température T2.

**Revendications**

1. Procédé pour caractériser et quantifier le carbone présent dans une formation superficielle, à partir d'un échantillon représentatif de ladite formation superficielle, **caractérisé en ce qu'**on applique au moins les étapes suivantes audit échantillon :

A. on chauffe ledit échantillon selon une première séquence de chauffe sous atmosphère inerte, et on mesure en continu une quantité de composés hydrocarbonés, une quantité de CO et une quantité de $CO_2$ libérés pendant ladite première séquence de chauffe, ladite première séquence de chauffe sous atmosphère inerte comprenant au moins une succession d'au moins six paliers isothermes d'une durée prédéterminée, ladite succession desdits au moins six paliers isothermes comprenant un premier palier isotherme à une première température comprise entre 80 et 200°C (T0), un deuxième palier isotherme à une deuxième température comprise entre 340 et 380°C (T1), un troisième palier isotherme à une troisième température comprise entre 400 et 440°C (T2), un quatrième palier isotherme à une quatrième température comprise entre 450 et 490°C (T3), un cinquième palier isotherme à une cinquième température comprise entre 500 et 540°C (T4), un sixième palier isotherme à une sixième température comprise entre 580 et 650°C (T5), lesdits paliers isothermes étant reliés entre eux par un gradient thermique ;

B. on chauffe un résidu dudit échantillon issu de ladite première séquence de chauffe selon une seconde séquence de chauffe sous atmosphère oxydante, et on mesure une quantité de CO et une quantité de $CO_2$ libérés pendant ladite seconde séquence de chauffe, ladite deuxième séquence de chauffe sous atmosphère oxydante débutant à une température minimale comprise entre 150 et 300°C, se terminant à une température maximale comprise entre 850 et 1200°C, et suivant un gradient thermique ;

C. on détermine une valeur d'un paramètre SCmin représentatif d'une proportion en carbone minéral par rapport au carbone total dudit échantillon à partir d'un ratio entre ladite quantité de $CO_2$ libéré par ledit résidu dudit échantillon au-delà d'une température intermédiaire de ladite deuxième séquence de chauffe comprise entre 620 et 680°C, et ladite quantité de $CO_2$ libéré par ledit résidu pendant ladite deuxième séquence de chauffe ;

D. on quantifie une teneur en carbone organique et/ou une teneur en carbone minéral dudit échantillon de la manière suivante :

i. si ladite valeur dudit paramètre SCmin est inférieure ou égale à une valeur seuil prédéfinie dudit paramètre SCmin, ladite teneur en carbone minéral est nulle et ladite teneur en carbone organique est fonction de la totalité desdites quantités de HC, de CO et de CO2 libérés pendant ladite première séquence de chauffe, et desdites quantités de CO et de CO2 libérés pendant ladite deuxième séquence de chauffe jusqu'à ladite température intermédiaire de ladite deuxième séquence de chauffe.

ii. si ladite valeur dudit paramètre SCmin est supérieure à ladite valeur seuil prédéfinie dudit paramètre SCmin :

- on estime une quantité de $CO_2$ libéré par craquage thermique de la matière organique dudit échantillon pendant ladite première séquence de chauffe au-delà de ladite troisième température (T2) à partir de ladite quantité de $CO_2$ libéré pendant ladite première séquence de chauffe jusqu'à ladite troisième température (T2) de ladite première séquence de chauffe, multipliée par un facteur k prédéfini ; et/ou
- on détermine ladite teneur en carbone organique et/ou ladite teneur en carbone minéral en fonction desdites quantités de HC, de CO et de $CO_2$ libérés pendant ladite première séquence de chauffe et desdites quantités de CO et de $CO_2$ libérés pendant ladite deuxième séquence de chauffe jusqu'à ladite température intermédiaire de ladite deuxième séquence de chauffe, et en prenant en compte ladite quantité estimée $CO_2$ libéré par craquage thermique de la matière organique dudit échantillon pendant ladite première séquence de chauffe au-delà de ladite troisième température (T2).

2. Procédé selon la revendication 1, dans lequel, si ladite valeur dudit paramètre SCmin est supérieure à ladite valeur seuil prédéfinie dudit paramètre SCmin :

- ladite teneur en carbone organique est fonction de la totalité de ladite quantité de HC libérés pendant ladite première séquence de chauffe, de ladite quantité de CO libéré pendant ladite première séquence de chauffe jusqu'à ladite cinquième température (T4) à laquelle on ajoute la moitié de ladite quantité de CO libéré pendant ladite première séquence de chauffe au-delà de ladite cinquième température (T4), de ladite quantité de $CO_2$ libéré pendant ladite première séquence de chauffe jusqu'à ladite troisième température (T2) à laquelle on ajoute ladite quantité estimée de $CO_2$ libéré par craquage thermique de la matière organique dudit échantillon pendant ladite première séquence de chauffe au-delà de ladite troisième température (T2), et desdites quantités de CO et de CO2 libérés pendant ladite deuxième séquence de chauffe jusqu'à ladite température intermédiaire de ladite deuxième séquence de chauffe, et/ou
- ladite teneur en carbone minéral est fonction de ladite quantité de $CO_2$ libéré pendant ladite première séquence de chauffe au-delà de ladite troisième température (T2) de ladite première séquence de chauffe à laquelle on retire ladite quantité estimée de $CO_2$ libéré par craquage thermique de la matière organique dudit échantillon pendant ladite première séquence de chauffe au-delà de ladite troisième température (T2), de ladite moitié de ladite quantité de CO libéré pendant ladite première séquence de chauffe au-delà de ladite cinquième température (T4), et de ladite quantité de $CO_2$ libéré pendant ladite deuxième séquence de chauffe au-delà de ladite température intermédiaire de ladite deuxième séquence de chauffe.

3. Procédé selon l'une des revendications 1 ou 2, dans lequel ledit facteur k prédéfini est compris entre 1.3 et 1.4, et vaut de préférence 1.3724.

4. Procédé selon l'une des revendications 1 ou 2, dans lequel, au moyen d'une pluralité d'échantillons provenant de formations superficielles non carbonatées, on applique au moins ladite première séquence de chauffe à chacun desdits échantillons de ladite pluralité d'échantillons, on mesure une quantité de $CO_2$ libéré par chacun desdits échantillons pendant ladite première séquence de chauffe, et on détermine ledit facteur k au moyen d'une régression linéaire appliquée entre lesdites quantités de $CO_2$ libérés par ladite pluralité d'échantillons jusqu'à ladite troisième température (T2) de ladite première séquence de chauffe et lesdites quantités de $CO_2$ libérés par ladite pluralité d'échantillons au-delà de ladite troisième température (T2) de ladite première séquence de chauffe.

5. Procédé selon l'une des revendications précédentes, dans lequel ladite valeur seuil prédéfinie dudit paramètre SCmin est comprise entre 0.03 et 0.05 et vaut de préférence 0.04.

6. Procédé selon l'une des revendications précédentes, dans lequel ladite première température (T0) de la dite première séquence de chauffe est comprise entre 80°C et 150°C, et vaut de préférence 150°C.

7. Procédé selon l'une des revendications précédentes, dans lequel ladite température minimale de ladite deuxième séquence de chauffe est égale à ladite première température (T0) de la dite première séquence de chauffe.

8. Procédé selon l'une des revendications précédentes, dans lequel ladite durée prédéterminée d'un desdits paliers isothermes de ladite première séquence de chauffe est comprise entre 3 et 5 minutes.

9. Procédé selon l'une des revendications précédentes, dans lequel l'un desdits gradients thermiques de ladite première séquence de chauffe est compris entre 1 et 50°C.min$^{-1}$, de préférence entre 20 et 25°C.min$^{-1}$.

10. Procédé selon l'une des revendications précédentes, dans lequel ledit gradient thermique de ladite deuxième séquence de chauffe est compris entre 20 et 40°C.min$^{-1}$, de préférence entre 20 et 25°C.min$^{-1}$.

11. Procédé selon l'une des revendications précédentes, dans lequel, si ladite valeur dudit paramètre SCmin est inférieure ou égale à ladite valeur seuil prédéfinie dudit paramètre SCmin :

   - ladite teneur en carbone minéral est définie par un paramètre MinCfs tel que MinCfs=0 ; et/ou
   - ladite teneur en carbone organique est définie par un paramètre TOCfs s'exprimant selon une formule du type :

$$TOC\text{fs} = (S2_t \; x \; 0.083) + (S3CO2_t \; x \frac{12}{440}) + (S3CO_t \; x \frac{12}{280}) + (S4CO2_a \; x \frac{12}{440}) +$$

$$(S4CO_a \; x \frac{12}{280})$$

où $S2_t$, $S3CO2_t$, $S3CO_t$ représentent lesdites quantités respectivement de HC, de $CO_2$ et de CO libérés pendant ladite première séquence de chauffe, $S4CO2_a$ et $S4CO_a$ représentent respectivement lesdites quantités de $CO_2$ et de CO libérés pendant ladite deuxième séquence de chauffe et jusqu'à ladite température intermédiaire de ladite deuxième séquence de chauffe.

12. Procédé selon l'une des revendications précédentes, dans lequel, si ladite valeur dudit paramètre SCmin est supérieure à ladite valeur seuil prédéfinie dudit paramètre SCmin :

   - ladite teneur en carbone minéral est définie par un paramètre MinCfs s'exprimant selon une formule du type :

$$MinC\text{fs} = ([S3CO2_b - S3CO2_c] x \frac{12}{440}) + (\tfrac{1}{2} S3CO_b \; x \frac{12}{280}) + (S4CO2_b \; x \frac{12}{440})$$

où $S3CO2_b$ représente ladite quantité de $CO_2$ libéré pendant ladite première séquence de chauffe au-delà de ladite troisième température (T2), $S3CO2_b$ représente ladite quantité estimée de $CO_2$ libéré par craquage thermique de la matière organique dudit échantillon pendant ladite première séquence de chauffe au-delà de ladite troisième température (T2), $S3CO_b$ représente ladite quantité de CO libéré pendant ladite première séquence de chauffe au-delà de ladite cinquième température (T4), et $S4CO2_b$ représente ladite quantité de $CO_2$ libéré pendant ladite deuxième séquence de chauffe au-delà de ladite température intermédiaire de ladite deuxième séquence de chauffe ; et/ou

- ladite teneur en carbone organique est définie par un paramètre TOCfs s'exprimant selon une formule du type :

$$TOC\text{fs} = (S2_t\, x\, 0.083) + \left([S3CO2_a + S3CO2_c]x\frac{12}{440}\right) + \left([S3CO_a + \tfrac{1}{2}\,S3CO_b]x\frac{12}{280}\right)$$

$$+ \left(S4CO2_a\, x\frac{12}{440}\right) + \left(S4CO_a\, x\frac{12}{280}\right)$$

où $S2t$ représente ladite quantité de HC libérés pendant ladite première séquence de chauffe, $S3CO2_a$ représente ladite quantité de $CO_2$ libéré pendant ladite première séquence de chauffe jusqu'à ladite troisième température (T2), $S3CO2_b$ représente ladite quantité estimée de $CO_2$ libéré par craquage thermique de la matière organique dudit échantillon pendant ladite première séquence de chauffe au-delà de ladite troisième température (T2), $S3CO_a$ et $S3CO_b$ représentent lesdites quantités de CO libérés pendant ladite première séquence de chauffe respectivement jusqu'à et au-delà de ladite cinquième température (T4), $S4CO2_a$ et $S4CO_a$ représentent respectivement lesdites quantités de $CO_2$ et de CO libérés pendant ladite deuxième séquence de chauffe et jusqu'à ladite température intermédiaire de ladite deuxième séquence de chauffe.

**Patentansprüche**

1. Verfahren zum Charakterisieren und Quantifizieren des Kohlenstoffs, welcher in einer oberflächennahen Formation vorliegt, ausgehend von einer Probe, die repräsentativ für die oberflächennahe Formation ist, **dadurch gekennzeichnet, dass** die Probe zumindest den folgenden Schritten unterzogen wird:

A. Erhitzen der Probe gemäß einer ersten Erhitzungssequenz unter Inertgasatmosphäre, und kontinuierliches Messen einer Menge an Kohlenwasserstoffverbindungen, einer Menge an CO und einer Menge an $CO_2$, wie sie während der ersten Erhitzungssequenz freigesetzt werden, wobei die erste Erhitzungssequenz unter Inertgasatmosphäre zumindest eine Abfolge von mindestens sechs isothermen Stufen von vorbestimmter Dauer umfasst, wobei die Abfolge der mindestens sechs isothermen Stufen eine erste isotherme Stufe bei einer ersten Temperatur im Bereich von 80 bis 200 °C (T0), eine zweite isotherme Stufe bei einer zweiten Temperatur im Bereich von 340 bis 380 °C (T1), eine dritte isotherme Stufe bei einer dritten Temperatur im Bereich von 400 bis 440 °C (T2), eine vierte isotherme Stufe bei einer vierten Temperatur im Bereich von 450 bis 490 °C (T3), eine fünfte isotherme Stufe bei einer fünften Temperatur im Bereich von 500 bis 540 °C (T4), eine sechste isotherme Stufe bei einer sechsten Temperatur im Bereich von 580 bis 650 °C (T5) umfasst, wobei die isothermen Stufen durch einen Temperaturgradienten miteinander verbunden sind;

B. Erhitzen eines Rückstands der Probe, der aus der ersten Erhitzungssequenz hervorgegangen ist, gemäß einer zweiten Erhitzungssequenz unter oxidierender Atmosphäre, und Messen einer Menge an CO und einer Menge an $CO_2$, wie sie während der zweiten Erhitzungssequenz freigesetzt werden, wobei die zweite Erhitzungssequenz unter oxidierender Atmosphäre bei einer Mindesttemperatur im Bereich von 150 bis 300 °C beginnt, bei einer Höchsttemperatur im Bereich von 850 bis 1200 °C endet und einem Temperaturgradienten folgt;

C. Bestimmen eines Wertes eines Parameters SCmin, welcher für einen Anteil an mineralischem Kohlenstoff bezogen auf den Gesamtkohlenstoff der Probe steht, ausgehend von einem Verhältnis zwischen der Menge an $CO_2$, die von dem Rückstand der Probe jenseits eines Temperaturzwischenwerts der zweiten Erhitzungssequenz freigesetzt wird, welcher im Bereich von 620 bis 680 °C liegt, und der Menge an $CO_2$, wie sie von dem Rückstand während der zweiten Erhitzungssequenz freigesetzt wird;

D. Quantifizieren eines Gehalts an organischem Kohlenstoff und/oder eines Gehalts an mineralischem Kohlenstoff der Probe auf folgende Art und Weise:

i. wenn der Wert des Parameters SCmin kleiner oder gleich einem vorbestimmten Schwellenwert für den Parameter SCmin ist, beträgt der Gehalt an mineralischem Kohlenstoff null, und der Gehalt an organischem Kohlenstoff hängt von der Gesamtheit der Mengen an HC, an CO und an CO2, wie sie während der ersten Erhitzungssequenz freigesetzt werden, und der Mengen an CO und an $CO_2$ ab, wie sie während der zweiten Erhitzungssequenz bis zu dem Zwischentemperaturwert der zweiten Erhitzungssequenz freigesetzt werden.

ii. wenn der Wert des Parameters SCmin höher als der vorbestimmte Schwellenwert für den Parameter SCmin ist:

- Schätzen einer Menge an $CO_2$, die durch thermisches Cracken der organischen Stoffe der Probe während der ersten Erhitzungssequenz jenseits der dritten Temperatur (T2) freigesetzt wird, ausgehend von der Menge an $CO_2$, die während der ersten Erhitzungssequenz bis zur dritten Temperatur (T2) der ersten Erhitzungssequenz freigesetzt wird, multipliziert mit einem vorbestimmten Faktor k; und/oder
- Bestimmen des Gehalts an organischem Kohlenstoff und/oder des Gehalts an mineralischem Kohlenstoff in Abhängigkeit von den Mengen an HC, an CO und an $CO_2$, wie sie während der ersten Erhitzungssequenz freigesetzt werden, und von den Mengen an CO und an $CO_2$, wie sie während der zweiten Erhitzungssequenz bis zum Zwischentemperaturwert der zweiten Erhitzungssequenz freigesetzt werden, sowie unter Berücksichtigung der geschätzten Menge an $CO_2$, diee durch thermisches Cracken der organischen Stoffe der Probe während der ersten Erhitzungssequenz jenseits der dritten Temperatur (T2) freigesetzt wird.

2. Verfahren nach Anspruch 1, wobei, wenn der Wert des Parameters Scmin höher als der vorbestimmte Schwellenwert für den Parameter SCmin ist:

- der Gehalt an organischem Kohlenstoff von der Gesamtheit der Menge an HC, welche während der ersten Erhitzungssequenz freigesetzt werden, der Menge an CO, die während der ersten Erhitzungssequenz bis zur fünften Temperatur (T4) freigesetzt wird, zuzüglich der Hälfte der Menge an an CO, die während der ersten Erhitzungssequenz jenseits der fünften Temperatur (T4) freigesetzt wird, der Menge an $CO_2$, die während der ersten Erhitzungssequenz bis zur dritten Temperatur (T2) freigesetzt wird, zuzüglich der geschätzten Menge an $CO_2$, die durch thermisches Cracken der organischen Stoffe der Probe während der ersten Erhitzungssequenz jenseits der dritten Temperatur (T2) freigesetzt wird, und von der Mengen an CO und an CO2 abhängt, wie sie während der zweiten Erhitzungssequenz bis zum Temperaturzwischenwert der zweiten Erhitzungssequenz freigesetzt werden, und/oder
- der Gehalt an mineralischem Kohlenstoff von der Menge an $CO_2$, die während der ersten Erhitzungssequenz jenseits der dritten Temperatur (T2) der ersten Erhitzungssequenz freigesetzt wird, abzüglich der geschätzten Menge an $CO_2$, die durch thermisches Cracken der organischen Stoffe der Probe während der ersten Erhitzungssequenz jenseits der dritten Temperatur (T2) freigesetzt wird, der Hälfte der Menge an CO, die während der ersten Erhitzungssequenz jenseits jenseits der fünften Temperatur (T4) freigesetzt wird, und von der Menge an $CO_2$ abhängt, die während der zweiten Erhitzungssequenz jenseits des Temperaturzwischenwerts der zweiten Erhitzungssequenz freigesetzt wird.

3. Verfahren nach einem der Ansprüche 1 oder 2, wobei der vorbestimmte Faktor im Bereich von 1,3 bis 1,4 liegt und vorzugsweise gleich 1,3724 ist.

4. Verfahren nach einem der Ansprüche 1 oder 2, wobei mittels einer Mehrzahl an Proben, die aus oberflächennahen, nicht carbonatisierten Formationen stammen, zumindest die erste Erhitzungssequenz auf jede der Proben der Mehrzahl an Proben angewendet wird, eine Menge an $CO_2$ gemessen wird, die von jeder der Proben während der ersten Erhitzungssequenz freigesetzt wird, und der Faktor k mittels einer Linearregression bestimmt wird, welche zwischen den Mengen an $CO_2$ angewendet wird, wie sie von der Mehrzahl an Proben bis zur dritten Temperatur (T2) der ersten Erhitzungssequenz freigesetzt werden, und den Mengen an $CO_2$ zur Anwendung gebracht wird, wie sie von der Mehrzahl an Proben jenseits der dritten Temperatur (T2) der ersten Erhitzungssequenz freigesetzt werden.

5. Verfahren nach einem einem der vorhergehenden Ansprüche, wobei der vorbestimmte Schwellenwert für den Parameter SCmin im Bereich von 0,03 bis 0,05 liegt und vorzugsweise gleich 0,04 ist.

6. Verfahren nach einem der vorhergehenden Ansprüche, wobei die erste Temperatur (T0) der ersten Erhitzungssequenz im Bereich von 80 °C bis 150 °C liegt und vorzugsweise gleich 150 °C ist.

7. Verfahren nach einem der vorhergehenden Ansprüche, wobei die Mindesttemperatur der zweiten Erhitzungssequenz gleich der ersten Temperatur (T0) der ersten Erhitzungssequenz ist.

8. Verfahren nach einem der vorhergehenden Ansprüche, wobei die vorbestimmte Dauer einer der isothermen Stufen der ersten Erhitzungssequenz im Bereich von 3 bis 5 Minuten liegt.

9. Verfahren nach einem der vorhergehenden Ansprüche, wobei einer der Temperaturgradienten der ersten Erhitzungssequenz im Bereich von 1 bis 50 °C.min$^{-1}$, vorzugsweise von 20 bis 25 °C.min$^{-1}$, liegt.

10. Verfahren nach einem der vorhergehenden Ansprüche, wobei der Temperaturgradient der zweiten Erhitzungssequenz im Bereich von 20 bis 40 °C.min$^{-1}$, vorzugsweise von 20 bis 25 °C.min$^{-1}$, liegt.

11. Verfahren nach einem der vorhergehenden Ansprüche, wobei, wenn der Wert des Parameters SCmin kleiner oder gleich dem vorbestimmten Schwellenwert für den Parameter SCmin ist:

- der Gehalt an mineralischem Kohlenstoff anhand eines Parameters MinCfs wie etwa MinCfs=0 definiert ist; und/oder
- der Gehalt an organischem Kohlenstoff anhand eines Parameters TOCfs definiert ist, welcher gemäß einer Formel folgender Art ausgedrückt wird:

$$TOC\text{fs} = (S2_{DE-DE} \; x \; 0{,}083) + \left(S3CO2_{DE-DE} \; x \; \frac{12}{440}\right) + \left(S3CO_{DE-DE} \; x \; \frac{12}{280}\right)$$
$$+ \left(S4CO2_a \; x \; \frac{12}{440}\right) + (S4CO_a \; x \; \frac{12}{280})$$

wobei $S2_t$, $S3CO2_t$, $S3COt$ für die Mengen an HC, an $CO_2$ beziehungsweise an CO stehen, wie sie während der ersten Erhitzungssequenz freigesetzt werden, $S4CO2_a$ und $S4CO_a$ für die Mengen an $CO_2$ beziehungsweise an CO stehen, wie sie während der zweiten Erhitzungssequenz und bis zum Zwischentemperaturwert der zweiten Erhitzungssequenz freigesetzt werden.

12. Verfahren nach einem der vorhergehenden Ansprüche, wobei, wenn der Wert des Parameters SCmin höher als der vorbestimmte Schwellenwert für den Parameter SCmin ist:

- der Gehalt an mineralischem Kohlenstoff anhand eines Parameters MinCfs definiert ist, welcher gemäß einer Formel folgender Art ausgedrückt wird:

$$MinC\text{fs} = ([S3CO2_b - S3CO2_c]x\frac{12}{440}) + (½ \; S3CO_b \; x \; \frac{12}{280})$$
$$+ (S4CO2_b \; x \; \frac{12}{440})$$

wobei $S3CO2_b$ für die Menge an $CO_2$ steht, die während der ersten Erhitzungssequenz jenseits der dritten Temperatur (T2) freigesetzt wird, $S3CO2_c$ für die geschätzte Menge an $CO_2$ steht, die durch thermisches Cracken der organischen Stoffe der Probe während der ersten Erhitzungssequenz jenseits der dritten Temperatur (T2) steht, $S3CO_b$ für die Menge an CO steht, die während der ersten Erhitzungssequenz jenseits der fünften Temperatur (T4) freigesetzt wird, und $S4CO2_b$ für die Menge an $CO_2$ steht, die während der zweiten Erhitzungssequenz jenseits des Zwischentemperaturwerts der zweiten Erhitzungssequenz freigesetzt wird; und/oder

- der Gehalt an organischem Kohlenstoff anhand eines Parameters TOCfs definiert ist, welcher gemäß einer Formel folgender Art ausgedrückt wird:

$$TOC\text{fs} = (S2_{DE-DE} \; x \; 0{,}083) + ([S3CO2_a + S3CO2_c]x\frac{12}{440}) + ([S3CO_a$$
$$+ ½S3CO_b]x\frac{12}{280}) + (S4CO2_a x\frac{12}{440}) + (S4CO_a x\frac{12}{280})$$

wobei $S2_t$ für die Menge an HC steht, welche während der ersten Erhitzungssequenz freigesetzt werden, $S3CO2_a$ für die Menge an $CO_2$ steht, die während der ersten Erhitzungssequenz bis zur dritten Temperatur (T2) freigesetzt wird, $S3CO2_c$ für die geschätzte Menge an $CO_2$ steht, die durch thermisches Cracken der organischen Stoffe der Probe während der ersten Erhitzungssequenz jenseits der dritten Temperatur (T2) freigesetzt wird, $S3CO_a$ und $S3CO_b$ für die Mengen an CO stehen, wie sie während der ersten Erhitzungssequenz bis zu beziehungsweise jenseits der fünften Temperatur (T4) freigesetzt werden, $S4CO2_a$ und $S4CO_a$ für die Mengen an $CO_2$ beziehungsweise CO stehen, wie sie während der zweiten Erhitzungssequenz und bis zum Zwischentemperaturwert der zweiten Erhitzungssequenz freigesetzt werden.

**Claims**

1. Method for characterizing and quantifying the carbon present in a surface formation, starting from a representative sample of said surface formation, **characterized in that** at least the following stages are applied to said sample:

   A. said sample is heated according to a first heating sequence under an inert atmosphere, and an amount of hydrocarbon compounds, an amount of CO and an amount of $CO_2$ which are released during said first heating sequence are continuously measured, said first heating sequence under an inert atmosphere comprising at least one sequence of at least six isothermal stationary phases of a predetermined duration, said sequence of said at least six isothermal stationary phases comprising a first isothermal stationary phase at a first temperature of between 80 and 200°C (T0), a second isothermal stationary phase at a second temperature of between 340 and 380°C (T1), a third isothermal stationary phase at a third temperature of between 400 and 440°C (T2), a fourth isothermal stationary phase at a fourth temperature of between 450 and 490°C (T3), a fifth isothermal stationary phase at a fifth temperature of between 500 and 540°C (T4) and a sixth isothermal stationary phase at a sixth temperature of between 580 and 650°C (T5), said isothermal stationary phases being interconnected by a thermal gradient;
   B. a residue of said sample resulting from said first heating sequence is heated according to a second heating sequence under an oxidizing atmosphere, and an amount of CO and an amount of $CO_2$ which are released during said second heating sequence are measured, said second heating sequence under an oxidizing atmosphere starting at a minimum temperature of between 150 and 300°C, ending at a maximum temperature of between 850 and 1200°C, and following a thermal gradient;
   C. a value of a parameter SCmin representative of a proportion of mineral carbon with respect to the total carbon of said sample is determined from a ratio of said amount of $CO_2$ released by said residue of said sample beyond an intermediate temperature of said second heating sequence of between 620 and 680°C to said amount of $CO_2$ released by said residue during said second heating sequence;
   D. an organic carbon content and/or a mineral carbon content of said sample is/are quantified in the following way:

      i. if said value of said parameter SCmin is less than or equal to a predefined threshold value of said parameter SCmin, said mineral carbon content is zero and said organic carbon content is a function of the totality of said amounts of HC, of CO and of $CO_2$ which are released during said first heating sequence, and of said amounts of CO and of $CO_2$ which are released during said second heating sequence up to said intermediate temperature of said second heating sequence,
      ii. if said value of said parameter SCmin is greater than said predefined threshold value of said parameter SCmin:

         - an amount of $CO_2$ released by thermal cracking of the organic matter of said sample during said first heating sequence beyond said third temperature (T2) is estimated from said amount of $CO_2$ released during said first heating sequence up to said third temperature (T2) of said first heating sequence, multiplied by a predefined factor k; and/or
         - said organic carbon content and/or said mineral carbon content is/are determined as a function of said amounts of HC, of CO and of $CO_2$ which are released during said first heating sequence and of said amounts of CO and of $CO_2$ which are released during said second heating sequence up to said intermediate temperature of said second heating sequence, and while taking into account said estimated amount of $CO_2$ released by thermal cracking of the organic matter of said sample during said first heating sequence beyond said third temperature (T2).

2. Method according to Claim 1, in which, if said value of said parameter SCmin is greater than said predefined threshold value of said parameter SCmin:

- said organic carbon content is a function of the totality of said amount of HC which are released during said first heating sequence, of said amount of CO released during said first heating sequence up to said fifth temperature (T4), to which is added half of said amount of CO released during said first heating sequence beyond said fifth temperature (T4), of said amount of $CO_2$ released during said first heating sequence up to said third temperature (T2), to which is added said estimated amount of $CO_2$ released by thermal cracking of the organic matter of said sample during said first heating sequence beyond said third temperature (T2), and of said amounts of CO and of $CO_2$ released during said second heating sequence up to said intermediate temperature of said second heating sequence, and/or

- said mineral carbon content is a function of said amount of $CO_2$ released during said first heating sequence beyond said third temperature (T2) of said first heating sequence, from which is withdrawn said estimated amount of $CO_2$ released by thermal cracking of the organic matter of said sample during said first heating sequence beyond said third temperature (T2), of said half of said amount of CO released during said first heating sequence beyond said fifth temperature (T4), and of said amount of $CO_2$ released during said second heating sequence beyond said intermediate temperature of said second heating sequence.

3. Method according to either of Claims 1 and 2, in which said predefined factor k is of between 1.3 and 1.4, and preferably has the value 1.3724.

4. Method according to either of Claims 1 and 2, in which, by means of a plurality of samples originating from non-carbonate surface formations, at least said first heating sequence is applied to each of said samples of said plurality of samples, an amount of $CO_2$ released by each of said samples during said first heating sequence is measured, and said factor k is determined by means of a linear regression applied between said amounts of $CO_2$ which are released by said plurality of samples up to said third temperature (T2) of said first heating sequence and said amounts of $CO_2$ which are released by said plurality of samples beyond said third temperature (T2) of said first heating sequence.

5. Method according to one of the preceding claims, in which said predefined threshold value of said parameter SCmin is of between 0.03 and 0.05, and preferably has the value 0.04.

6. Method according to one of the preceding claims, in which said first temperature (T0) of said first heating sequence is of between 80°C and 150°C, and preferably has the value 150°C.

7. Method according to one of the preceding claims, in which said minimum temperature of said second heating sequence is equal to said first temperature (T0) of said first heating sequence.

8. Method according to one of the preceding claims, in which said predetermined duration of one of said isothermal stationary phases of said first heating sequence is of between 3 and 5 minutes.

9. Method according to one of the preceding claims, in which one of said thermal gradients of said first heating sequence is of between 1 and 50°C.min$^{-1}$, preferably between 20 and 25°C.min$^{-1}$.

10. Method according to one of the preceding claims, in which said thermal gradient of said second heating sequence is of between 20 and 40°C.min$^{-1}$, preferably between 20 and 25°C.min$^{-1}$.

11. Method according to one of the preceding claims, in which, if said value of said parameter SCmin is less than or equal to said predefined threshold value of said parameter SCmin:

- said mineral carbon content is defined by a parameter MinCfs such that MinCfs = 0; and/or
- said organic carbon content is defined by a parameter TOCfs which is expressed according to a formula of the type:

$$TOCfs = (S2_t \ x \ 0.083) + \left(S3CO2_t \ x \ \frac{12}{440}\right) + \left(S3CO_t \ x \ \frac{12}{280}\right) + \left(S4CO2_a \ x \ \frac{12}{440}\right) + \left(S4CO_a \ x \ \frac{12}{280}\right)$$

where $S2_t$, $S3CO2t$, $S3CO_t$ represent said amounts respectively of HC, of $CO_2$ and of CO which are released during said first heating sequence, $S4CO2_a$ and $S4CO_a$ respectively represent said amounts of $CO_2$ and of CO which are released during said second heating sequence and up to said intermediate temperature of said second heating sequence.

12. Method according to one of the preceding claims, in which, if said value of said parameter SCmin is greater than said predefined threshold value of said parameter SCmin:

- said mineral carbon content is defined by a parameter MinCfs which is expressed according to a formula of the type:

$$MinCfs = ([S3CO2_b - S3CO2_c] x \frac{12}{440}) + (\frac{1}{2} S3CO_b \ x \ \frac{12}{280}) + (S4CO2_b \ x \ \frac{12}{440})$$

in which $S3CO2_b$ represents said amount of $CO_2$ released during said first heating sequence beyond said third temperature (T2), $S3CO2_c$ represents said estimated amount of $CO_2$ released by thermal cracking of the organic matter of said sample during said first heating sequence beyond said third temperature (T2), $S3CO_b$ represents said amount of CO released during said first heating sequence beyond said fifth temperature (T4), and $S4CO2_b$ represents said amount of $CO_2$ released during said second heating sequence beyond said intermediate temperature of said second heating sequence; and/or

- said organic carbon content is defined by a parameter TOCfs which is expressed according to a formula of the type:

$$TOCfs = (S2_t \ x \ 0.083) + ([S3CO2_a + S3CO2_c] x \frac{12}{440}) + ([S3CO_a + \frac{1}{2}S3CO_b] x \frac{12}{280}) + (S4CO2_a x \frac{12}{440}) + (S4CO_a x \frac{12}{280})$$

where $S2_t$ represents said amount of HC which are released during said first heating sequence, $S3CO2_a$ represents said amount of $CO_2$ released during said first heating sequence up to said third temperature (T2), $S3CO2_c$ represents said estimated amount of $CO_2$ released by thermal cracking of the organic matter of said sample during said first heating sequence beyond said third temperature (T2), $S3CO_a$ and $S3CO_b$ represent said amounts of CO which are released during said first heating sequence respectively up to and beyond said fifth temperature (T4), $S4CO2_a$ and $S4CO_a$ respectively represent said amounts of $CO_2$ and of CO which are released during said second heating sequence and up to said intermediate temperature of said second heating sequence.

[Fig. 1]

[Fig. 2A]

[Fig. 2B]

[Fig. 2C]

[Fig. 2D]

[Fig. 3A]

[Fig. 3B]

[Fig. 4A]

[Fig. 4B]

[Fig. 5A]

[Fig. 5B]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2227797
- US 3953171 A
- FR 2472754
- US 4352673 A
- FR 3072173

**Littérature non-brevet citée dans la description**

- **BEHAR F.** ; **BEAUMONT V.** ; **DE B.** ; **PENTEADO H.L.** *Rock-Eval 6 Technology: Performances and Developments, Oil & Gas Science and Technology*, 2001, vol. 56, 111-134
- **DISNAR, J.R.** ; **GUILLET, B.** ; **KERAVIS, D.** ; **DI-GIOVANNI, C.** ; **SEBAG, D.** Soil organic matter (SOM) characterization by Rock-Eval pyrolysis: scope and limitations.. *Organic Geochemistry*, 2003, vol. 34, 327-343
- **MALOU, O.P.** ; **SEBAG, D.** ; **MOULIN P.** ; **CHEVAL-LIER, T.** ; **BADIANE-NDOUR, N.Y.** ; **THIAM, A.** ; **CHAPUIS-LARDY, L.** The Rock-Eval® signature of soil organic carbon in Arenosols of the Senegalese groundnut Basin. How do agricultural practices matter?. *Agriculture, Ecosystems & Environment*, 2020, vol. 301, 107030, https://doi.org/10.1016/j.agee.2020.107030
- **PILLOT, D.** ; **DEVILLE, E.** ; **PRINZHOFER, A.** Identification and Quantification of Carbonate Species Using Rock-Eval Pyrolysis.. *Oil & Gas Science and Technology - Revue d'IFP Energies nouvelles*, 2014, vol. 69, 341-349
- **SEBAG, D.** ; **DISNAR, J.R.** ; **GUILLET, B.** ; **DI GIOVANNI, C.** ; **VERRECCHIA, E.P.** ; **DURAND, A.** Monitoring organic matter dynamics in soil profiles by ''Rock-Eval pyrolysis'': bulk characterization and quantification of degradation.. *European Journal of Soil Science*, 2006, vol. 57, 344-355
- **SEBAG, D.** ; **VERRECCHIA, E.P.** ; **CÉCILLON, L.** ; **ADATTE, T.** ; **ALBRECHT, R.** ; **AUBERT, M.** ; **BUREAU, F.** ; **CAILLEAU, G.** ; **COPARD, Y.** ; **DECAËNS, T.** Dynamics of soil organic matter based on new Rock-Eval indices.. *Geoderma*, 2016, vol. 284, 185-203